# EUROPEAN PATENT SPECIFICATION

(11) **EP 2 138 510 B1**
(45) Date of publication and mention of the grant of the patent: **21.08.2013**
(21) Application number: 09163836.1
(22) Date of filing: 27.07.2000
(51) Int. Cl.: C07K 14/54

(54) **Methods of generating antibodies against cytokines**
Methoden der Erzeugung von gegen Zytokine gerichteten Antikörpern
Méthodes de génération d'anticorps dirigés contre des cytokines

(30) Priority: 30.07.1999 US 364674; 06.08.1999 US 369643
(43) Date of publication of application: 30.12.2009
(62) Divisional of application: 00950787.2
(73) Proprietor: Merck Sharp & Dohme Corp., Rahway, NJ 07065-0907 (US)
(72) Inventor: Timans, Jacqueline C., Mountain View, CA 94043 (US); Kastelein, Robert A., Portola Valley, CA 94028 (US); Bazan, Fernando J., Menlo Park, CA 94025 (US)
(74) Representative: Horgan, James Michael Frederic

(56) References cited:
- PFLANZ S ET AL: "IL-27 a heterodimetric cytokine composed of EB13 and p28 protein, induces proliferation of native CD4+ T cells" IMMUNITY, CELL PRESS, US, vol. 16, 1 June 2002 (2002-06-01), pages 779-790, XP002982845 ISSN: 1074-7613
- GATELY M K ET AL: "THE INTERLEUKIN-12/ INTERLEUKIN-12-RECEPTOR SYSTEM: ROLE IN NORMAL AND PATHOLOGIC IMMUNE RESPONSES" ANNUAL REVIEW OF IMMUNOLOGY, ANNUAL REVIEWS INC, US, vol. 16, 1 January 1998 (1998-01-01), pages 495-521, XP008024350 ISSN: 0732-0582

## Description

### FIELD OF THE INVENTION

The present invention pertains to compositions related to proteins which function in controlling biology and physiology of mammalian cells, e.g., cells of a mammalian immune system. In particular, it provides methods of making antibodies useful, e.g., to regulate activation, development, differentiation, and function of various cell types, including hematopoietic cells.

### BACKGROUND OF THE INVENTION

Recombinant DNA technology refers generally to the technique of integrating genetic information from a donor source into vectors for subsequent processing, such as through introduction into a host, whereby the transferred genetic information is copied and/or expressed in the new environment. Commonly, the genetic information exists in the form of complementary DNA (cDNA) derived from messenger RNA (mRNA) coding for a desired protein product. The carrier is frequently a plasmid having the capacity to incorporate cDNA for later replication in a host and, in some cases, actually to control expression of the cDNA and thereby direct synthesis of the encoded product in the host.

For some time, it has been known that the mammalian immune response is based on a series of complex cellular interactions, called the "immune network". Recent research has provided new insights into the inner workings of this network. While it remains clear that much of the response does, in fact, revolve around the network-like interactions of lymphocytes, macrophages, granulocytes, and other cells, immunologists now generally hold the opinion that soluble proteins, known as lymphokines, cytokines, or monokines, play a critical role in controlling these cellular interactions. Thus, there is considerable interest in the isolation, characterization, and mechanisms of action of cell modulatory factors, an understanding of which will lead to significant advancements in the diagnosis and therapy of numerous medical abnormalities, e.g., immune system disorders. Some of these factors are hematopoietic growth and/or differentiation factors, e.g., stem cell factor (SCF) or IL-11. See, e.g., Mire-Sluis and Thorpe (1998) Cytokines Academic Press, San Diego; Thomson (ed. 1998) The Cytokine Handbook (3d ed.) Academic Press, San Diego; Metcalf and Nicola (1995) The Hematopoietic Colony Stimulating Factors Cambridge University Press; and Aggarwal and Gutterman (1991) Human Cytokines Blackwell.

Lymphokines apparently mediate cellular activities in a variety of ways. They have been shown to support the proliferation, growth, and differentiation of pluripotential hematopoietic stem cells into vast numbers of progenitors comprising diverse cellular lineages making up a complex immune system. Proper and balanced interactions between the cellular components are necessary for a healthy immune response. The different cellular lineages often respond in a different manner when lymphokines are administered in conjunction with other agents.

Cell lineages especially important to the immune response include two classes of lymphocytes: B-cells, which can produce and secrete immunoglobulins (proteins with the capability of recognizing and binding to foreign matter to effect its removal), and T-cells of various subsets that secrete lymphokines and induce or suppress the B-cells and various other cells (including other T-cells) making up the immune network. These lymphocytes interact with many other cell types.

Another important cell lineage is the mast cell (which has not been positively identified in all mammalian species), which is a granule-containing connective tissue cell located proximal to capillaries throughout the body. These cells are found in especially high concentrations in the lungs, skin, and gastrointestinal and genitourinary tracts. Mast cells play a central role in allergy-related disorders, particularly anaphylaxis as follows: when selected antigens crosslink one class of immunoglobulins bound to receptors on the mast cell surface, the mast cell degranulates and releases mediators, e.g., histamine, serotonin, heparin, and prostaglandins, which cause allergic reactions, e.g., anaphylaxis.

Research to better understand and treat various immune disorders has been hampered by the general inability to maintain cells of the immune system in vitro. Immunologists have discovered that culturing these cells can be accomplished through the use of T-cell and other cell supernatants, which contain various growth factors, including many of the lymphokines.

From the foregoing, it is evident that the discovery and development of new lymphokines, e.g., related to IL-11, could contribute to new therapies for a wide range of degenerative or abnormal conditions which directly or indirectly involve the immune system and/or hematopoietic cells. In particular, the discovery and development of lymphokines which enhance or potentiate the beneficial activities of known lymphokines would be highly advantageous.

### SUMMARY OF THE INVENTION

The present invention is defined by the claims.

The present invention is based, in part, upon the discovery of new cytokine sequences exhibiting significant sequence and structural similarity to IL-11. In particular, it provides primate, e.g., human, and rodent, e.g., mouse, sequences. Functional equivalents exhibiting significant sequence homology will be available from other mammalian, e.g., cow, horse, and rat, mouse, and non-mammalian species.

### DETAILED DESCRIPTION OF THE PREFERRED EMBODIMENTS

### I. General

The present invention relates to amino acid sequences which are cytokines, e.g., which are secreted molecules which can mediate a signal between immune or other cells. See, e.g., Paul (1997) Fundamental Immunology (3d ed.) Raven Press, N.Y. The full length cytokines, and fragments, or antagonists will be useful in physiological modulation of cells expressing a receptor. It is likely that IL-D80 has either stimulatory or inhibitory effects on hematopoietic cells, including, e.g., lymphoid cells, such as T-cells, B-cells, natural killer (NK) cells, macrophages, dendritic cells, hematopoietic progenitors, etc. The proteins are useful as antigens, e.g., immunogens, for raising antibodies to various epitopes on the protein, both linear and conformational epitopes.

A cDNA encoding IL-D80 was identified from various primate, e.g., human, sequences of BACs of Chromosome 16. See, e.g., CIT987SK-A-575C2, and CIT987SK-A-761H5. The molecule was designated huIL-D80. A human EST has been identified and described, human EST AI085007. A mouse EST AA266872 has also been identified and described.

The primate, e.g., human, gene will encode a small soluble cytokine-like protein, of about 216 amino acids (for SEQ ID NO:2) or about 243 amino acids (for SEQ ID NO: 8). See Table 1 and SEQ. ID. NOs: 1, 2, 7, and 8. IL-D80 exhibits structural motifs characteristic of a member of the long chain cytokines. Compare, e.g., IL-D80 and IL-11, sequences available from GenBank. See also rodent sequences and Table 2 or 3.

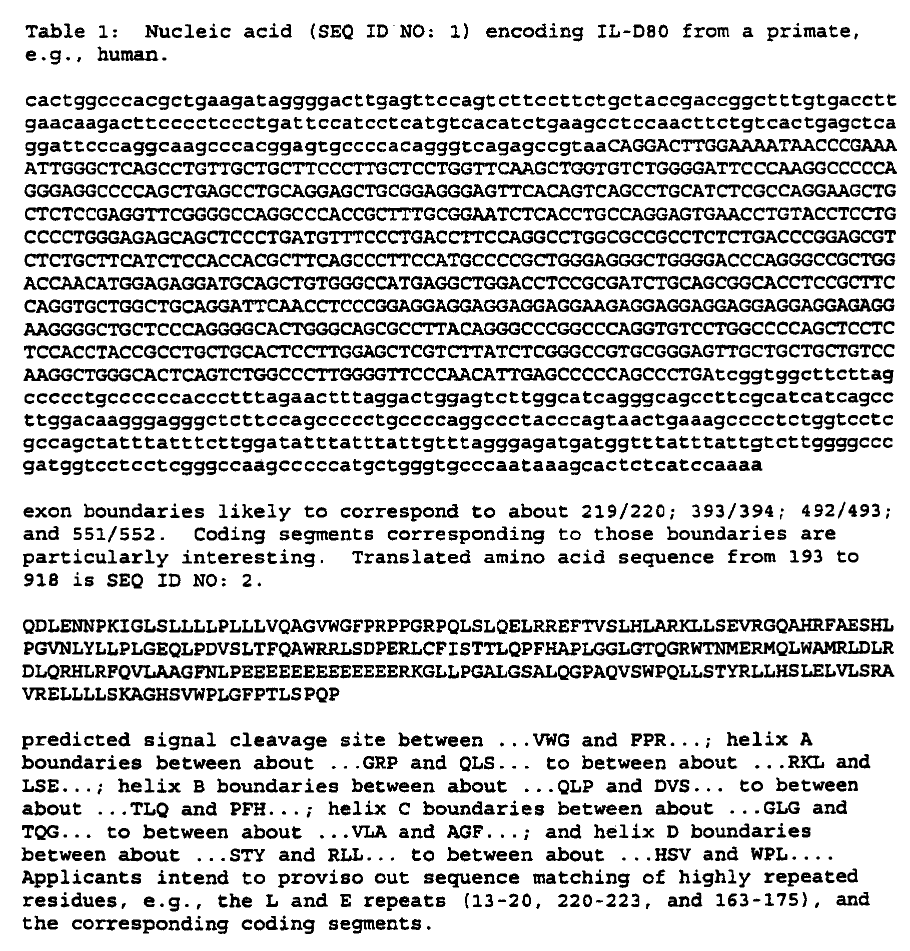

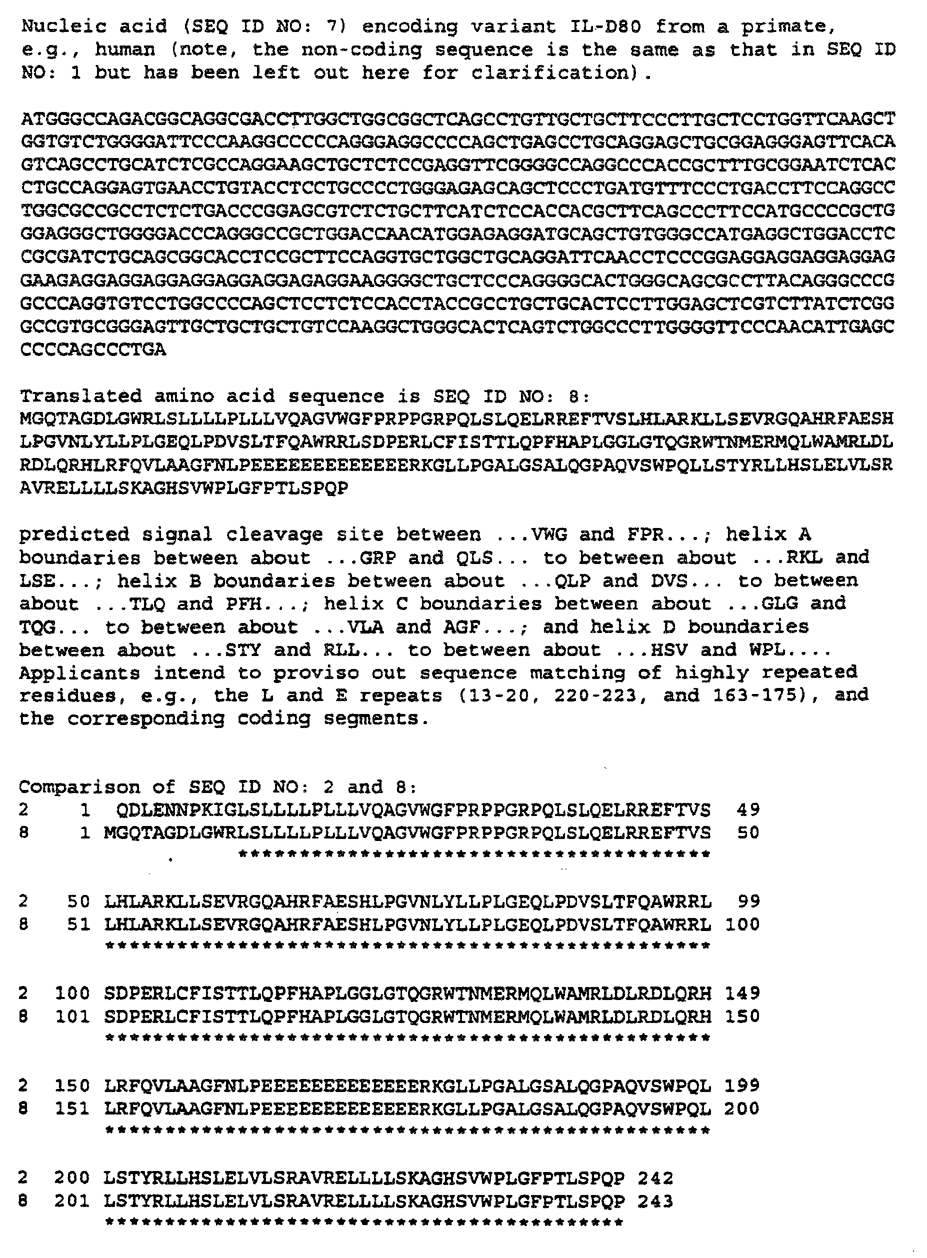

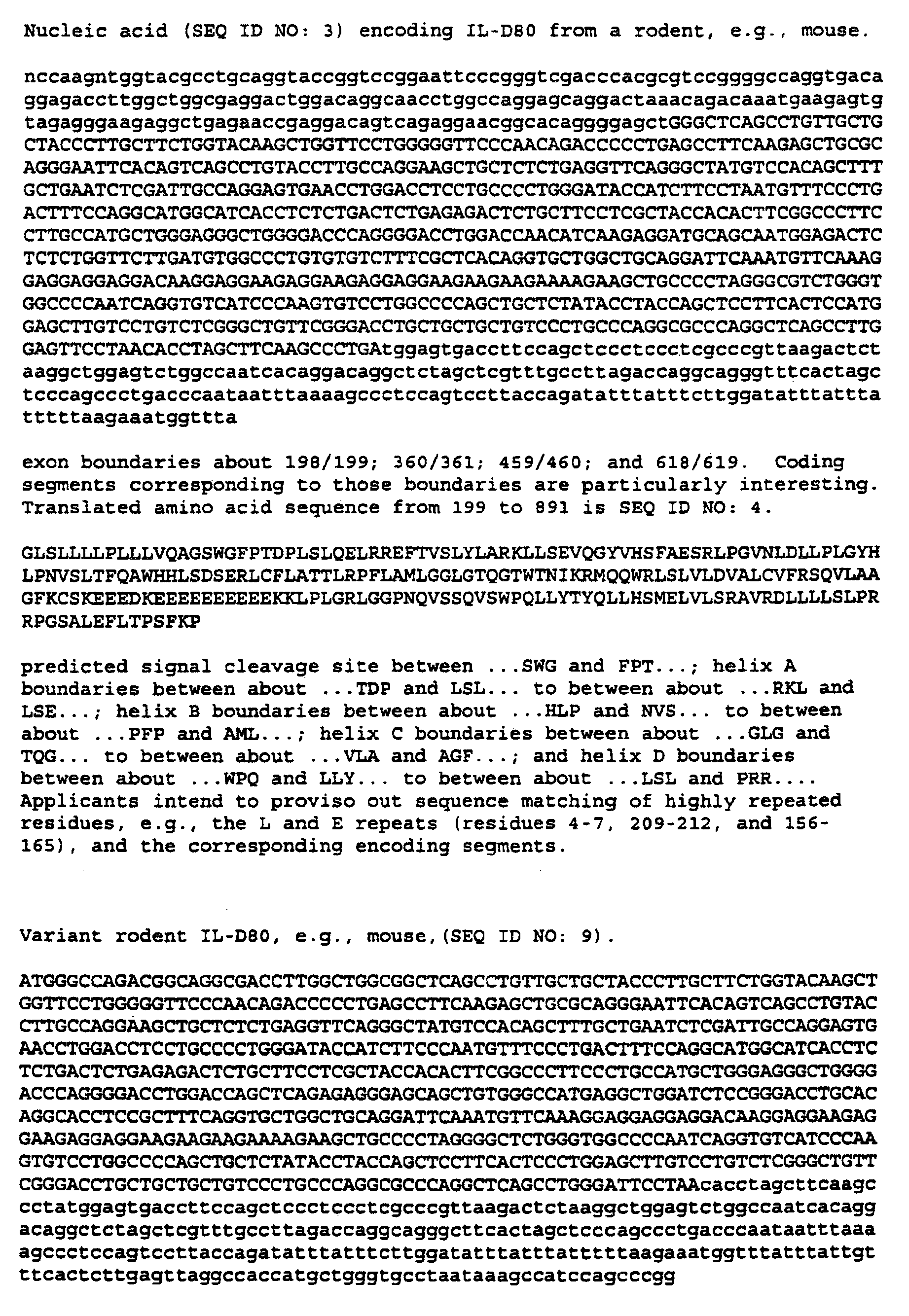

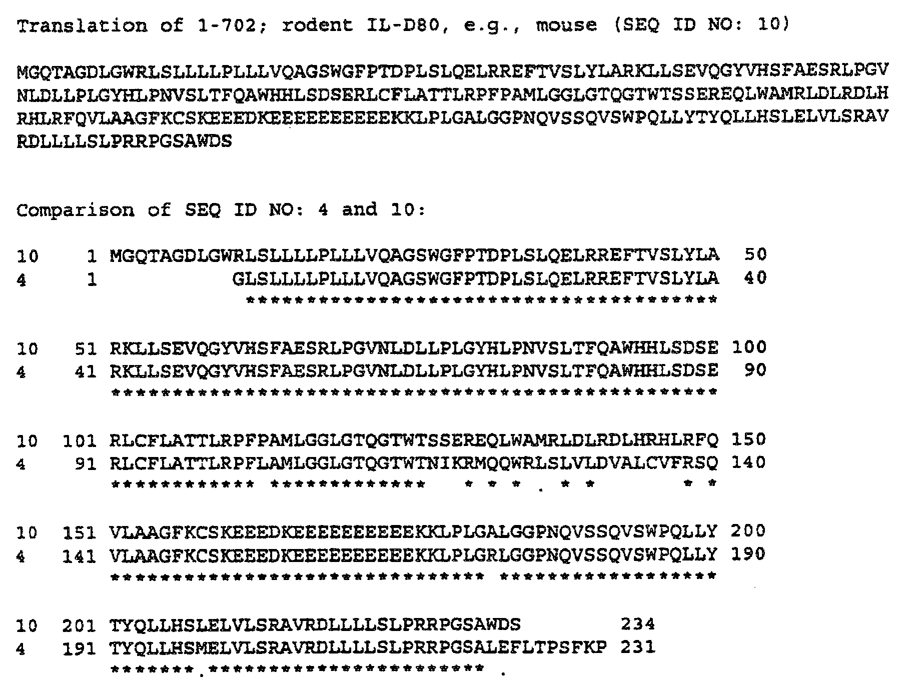

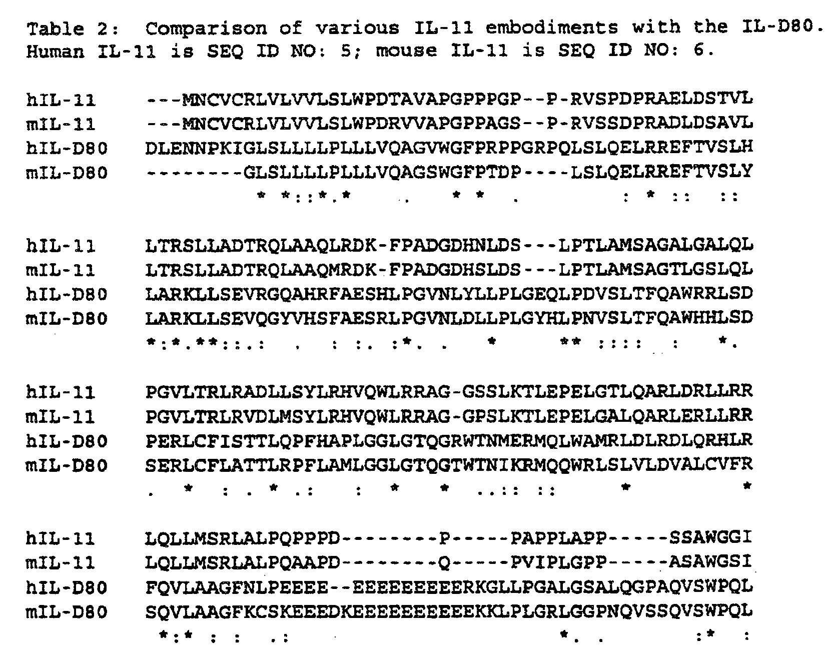

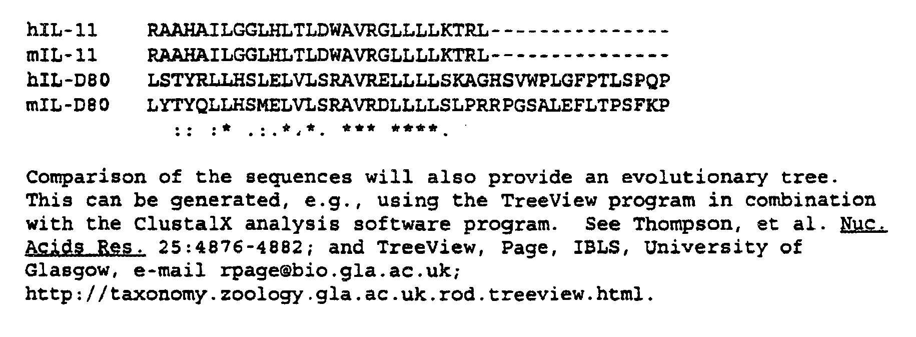

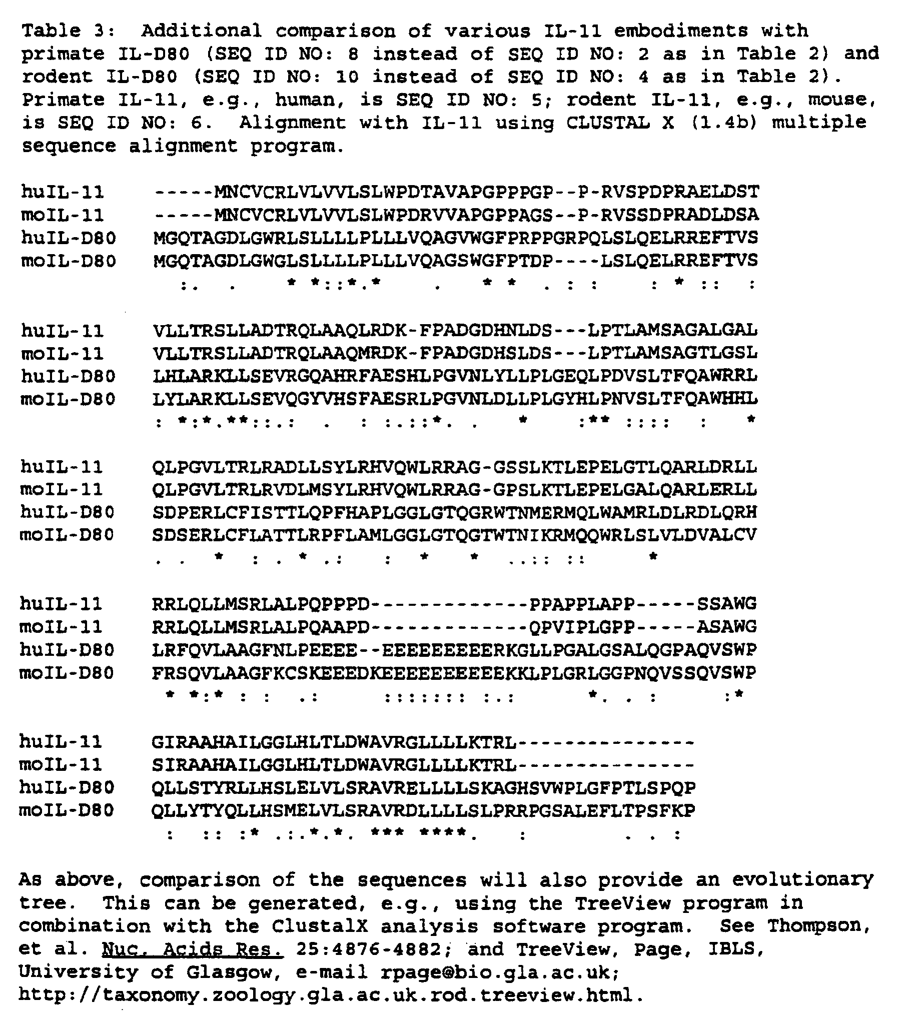

The structural homology of IL-D80 to related cytokine proteins suggests related function of this molecule. IL-D80 is a long chain cytokine exhibiting sequence similarity to IL-11.

Many aspects of the biology of IL-11 are well recognized. See, e.g., Sonis, et al. (1999) Leukemia 13:831-834; Jacques, et al. (1998) Res. Immunol. 149:737-740; Trepicchio, et al. (1998) Ann. N.Y. Acad. Sci. 856:12-21; Jacobsen (1998) in Thomson The cytokine Handbook, Academic Press; Maslak, et al. (1998) Semin. Hematol. 35:253-260; Leng, et al. (1997) Int. J. Biochem. Cell. Biol. 29:1059-1062; Du, et al. (1997) Blood 89:3897-3908; Goldman (1995) Stem Cells 13:462-471; and Du, et al. (1995) Curr. Opin. Hematol. 2:182-188. The biology of the IL-D80 is expected to be similar, e.g., some of the biological activities may overlap.

IL-D80 agonists, or antagonists, may also act as functional or receptor antagonists, e.g., which block IL-11 binding to its respective receptors, or mediating the opposite actions. Thus, IL-D80, or its antagonists, may be useful in the treatment of abnormal medical conditions, including immune disorders, e.g., T cell immune deficiencies, chronic inflammation, or tissue rejection, or in cardiovascular or neurophysiological conditions. Compositions combining the IL-D80 and IL-11 related reagents will often be used.

The natural antigens are capable of mediating various biochemical responses which lead to biological or physiological responses in target cells. The preferred embodiment characterized herein is from human, but other primate, or other species counterparts exist in nature. Additional sequences for proteins in other mammalian species, e.g., primates, canines, felines, and rodents, should also be available, particularly the domestic animal species. See below. The descriptions below are directed, for exemplary purposes, to a human IL-D80, but are likewise applicable to related embodiments from other species.

### II. Purified IL-D80

Primate, e.g., human, IL-D80 amino acid sequence, is shown in several embodiments, e.g., SEQ ID NO: 2, 4, 8, or 10. Other naturally occurring nucleic acids which encode the protein can be isolated by standard procedures using the provided sequence, e.g., PCR techniques, or by hybridization. These amino acid sequences, provided amino to carboxy, are important in providing sequence information for the cytokine allowing for distinguishing the protein antigen from other proteins and exemplifying numerous variants. Moreover, the peptide sequences allow preparation of peptides to generate antibodies to recognize such segments, and nucleotide sequences allow preparation of oligonucleotide probes, both of which are strategies for detection or isolation, e.g., cloning, of genes encoding such sequences.

As used herein, the term "human soluble IL-D80" shall encompass, when used in a protein context, a protein having amino acid sequence corresponding to a soluble polypeptide shown in SEQ ID NO: 2, or 8, or significant fragments thereof. Preferred embodiments comprise a plurality of distinct, e.g., nonoverlapping, segments of the specified length. Typically, the plurality will be at least two, more usually at least three, and preferably 5, 7, or even more. While the length minima are provided, longer lengths, of various sizes, may be appropriate, e.g., one of length 7, and two of length 12.

Binding components, e.g., antibodies, typically bind to an IL-D80 with high affinity, e.g., at least about 100 nM, usually better than about 30 nM, preferably better than about 10 nM, and more preferably at better than about 3 nM. Counterpart proteins will be found in mammalian species other than human, e.g., other primates, ungulates, or rodents. Non-mammalian species should also possess structurally or functionally related genes and proteins, e.g., birds or amphibians.

The term "polypeptides" as used herein includes a significant fragment or segment, and encompasses a stretch of amino acid residues of at least about 8 amino acids, generally at least about 12 amino acids, typically at least about 16 amino acids, preferably at least about 20 amino acids, and, in particularly preferred embodiments, at least about 30 or more amino acids, e.g., 35, 40, 45, 50, 60, 75, 100, etc. Such fragments may have ends which begin and/or end at virtually all positions, e.g., beginning at residues 1, 2, 3, etc., and ending at, e.g., 150, 149, 148, etc., in all practical combinations. Particularly interesting peptides have ends corresponding to structural domain boundaries, e.g., helices A, B, C, and/or D. See Tables 1, 2, and 3.

The term "blinding composition" refers to molecules that bind with specificity to IL-D80, e.g., in an antibody-antigen interaction. The specificity may be more or less inclusive, e.g., specific to a particular embodiment, or to groups of related embodiments, e.g., primate, rodent, etc. It also includes compounds, e.g., proteins, which specifically associate with IL-D80, including in a natural physiologically relevant protein-protein interaction, either covalent or non-covalent. The molecule may be a polymer, or chemical reagent. A functional analog may be a protein with structural modifications, or it may be a molecule which has a molecular shape which interacts with the appropriate binding determinants. The compounds may serve as agonists or antagonists of a receptor binding interaction, see, e.g., Goodman, et al. (eds.) Goodman & Gilman's: The Pharmacological Bases of Therapeutics (current ed.) Pergamon Press.

Substantially pure, e.g., in a protein context, typically means that the protein is free from other contaminating proteins, nucleic acids, or other biologicals derived from the original source organism. Purity may be assayed by standard methods, typically by weight, and will ordinarily be at least about 40% pure, generally at least about 50% pure, often at least about 60% pure, typically at least about 80% pure, preferably at least about 90% pure, or, at least about 95% pure. Carriers or excipients will often be added.

Solubility of a polypeptide or fragment depends upon the environment and the polypeptide. Many parameters affect polypeptide solubility, including temperature, electrolyte environment, size and molecular characteristics of the polypeptide, and nature of the solvent. Typically, the temperature at which the polypeptide is used ranges from about 4° C to about 65° C. Usually the temperature at use is greater than about 18° C. For diagnostic purposes, the temperature will usually be about room temperature or warmer, but less than the denaturation temperature of components in the assay. For therapeutic purposes, the temperature will usually be body temperature, typically about 37° C for humans and mice, though under certain situations the temperature may be raised or lowered in situ or in vitro.

The size and structure of the polypeptide should generally be in a substantially stable state, and usually not in a denatured state. The polypeptide may be associated with other polypeptides in a quaternary structure, e.g., to confer solubility, or associated with lipids or detergents.

The solvent and electrolytes will usually be a biologically compatible buffer, of a type used for preservation of biological activities, and will usually approximate a physiological aqueous solvent. Usually the solvent will have a neutral pH, typically between about 5 and 10, and preferably about 7.5. On some occasions, one or more detergents will be added, typically a mild non-denaturing one, e.g., CHS (cholesteryl hemisuccinate) or CHAPS (3-[3-cholamidopropyl)dimethylammonio]-1-propane sulfonate), or a low enough concentration as to avoid significant disruption of structural or physiological properties of the protein. In other instances, a harsh detergent may be used to effect significant denaturation.

### III. Physical Variants

This invention also relates to proteins or peptides having substantial amino acid sequence identity with the amino acid sequence of the IL-D80 antigen. The variants include species, polymorphic, or allelic variants.

Amino acid sequence homology, or sequence identity, is determined by optimizing residue matches, if necessary, by introducing gaps as required. See also Needleham, et al. (1970) J. Mol. Biol. 48:443-453; Sankoff, et al. (1983) Chapter One in Time Warps, String Edits, and Macromolecules: The Theory and Practice of Sequence Comparison, Addison-Wesley, Reading, MA; and software packages from IntelliGenetics, Mountain View, CA; and the University of Wisconsin Genetics Computer Group, Madison, WI. Sequence identity changes when considering conservative substitutions as matches. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. The conservation may apply to biological features, functional features, or structural features. Homologous amino acid sequences are typically intended to include natural polymorphic or allelic and interspecies variations of a protein sequence. Typical homologous proteins or peptides will have from 25-100% identity (if gaps can be introduced), to 50-100% identity (if conservative substitutions are included) with the amino acid sequence of the IL-D80. Identity measures will be at least about 35%, generally at least about 40%, often at least about 50%, typically at least about 60%, usually at least about 70%, preferably at least about 80%, and more preferably at least about 90%.

The isolated IL-D80 DNA can be readily modified by nucleotide substitutions, nucleotide deletions, nucleotide insertions, and inversions of short nucleotide stretches. These modifications result in novel DNA sequences which encode these antigens, their derivatives, or proteins having similar physiological, immunogenic, antigenic, or other functional activity. These modified sequences can be used to produce mutant antigens or to enhance expression. Enhanced expression may involve gene amplification, increased transcription, increased translation, and other mechanisms. "Mutant IL-D80" encompasses a polypeptide otherwise falling within the sequence identity definition of the IL-D80 as set forth above, but having an amino acid sequence which differs from that of IL-D80 as normally found in nature, whether by way of deletion, substitution, or insertion. This generally includes proteins having significant identity with a protein having sequence of SEQ ID NO: 2, 4, 8, or 10, and as sharing various biological activities, e.g., antigenic or immunogenic, with those sequences, and in preferred embodiments contain most of the natural full length disclosed sequences. Full length sequences will typically be preferred, though truncated versions will also be useful, likewise, genes or proteins found from natural sources are typically most desired. Similar concepts apply to different IL-D80 proteins, particularly those found in various warm blooded animals, e.g., mammals and birds. These descriptions are generally meant to encompass many IL-D80 proteins, not limited to the particular primate embodiments specifically discussed.

IL-D80 mutagenesis can also be conducted by making amino acid insertions or deletions. Substitutions, deletions, insertions, or any combinations may be generated to arrive at a final construct. Insertions include amino-or carboxy- terminal fusions. Random mutagenesis can be conducted at a target codon and the expressed mutants can then be screened for the desired activity. Methods for making substitution mutations at predetermined sites in DNA having a known sequence are well known in the art, e.g., by M13 primer mutagenesis or polymerase chain reaction (PCR) techniques. See, e.g., Sambrook, et al. (1989); Ausubel, et al. (1987 and Supplements); and Kunkel, et al. (1987) Methods in Enzymol. 154:367-382. Preferred embodiments include, e.g., 1-fold, 2-fold, 3-fold, 5-fold, 7-fold, etc., preferably conservative substitutions at the nucleotide or amino acid levels. Preferably the substitutions will be away from the conserved cysteines, and often will be in the regions away from the helical structural domains. Such variants may be useful to produce specific antibodies, and often will share many or all biological properties.

The present invention also relates to recombinant proteins, e.g., heterologous fusion proteins using segments from these proteins. A heterologous fusion protein is a fusion of proteins or segments which are naturally not normally fused in the same manner. A similar concept applies to heterologous nucleic acid sequences.

In addition, new constructs may be made from combining similar functional domains from other proteins. For example, target-binding or other segments may be "swapped" between different new fusion polypeptides or fragments. See, e.g., Cunningham, et al. (1989) Science 243:1330-1336; and O'Dowd, et al. (1988) J. Biol. Chem. 263:15985-15992.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence, e.g., PCR techniques. Structural analysis can be applied to this gene, in comparison to the IL-11 family of cytokines. Alignment of the human IL-D80 sequences with other members of the IL-11 family should allow definition of structural features. In particular, β-sheet and α-helix residues can be determined using, e.g., RASMOL program, see Bazan, et al. (1996) Nature 379:591; Lodi, et al. (1994) Science 263:1762-1766; Sayle and Milner-White (1995) TIBS 20:374-376; and Gronenberg, et al. (1991) Protein Engineering 4:263-269. Preferred residues for substitutions include the surface exposed residues which would be predicted to interact with receptor. Other residues which should conserve function will be conservative substitutions, particularly at position far from the surface exposed residues.

### IV. Functional Variants

The blocking of physiological response to IL-D80s may result from the competitive inhibition of binding of the ligand to its receptor.

In vitro assays will often use isolated protein, soluble fragments comprising receptor binding segments of these proteins, or fragments attached to solid phase substrates. These assays will also allow for the diagnostic determination of the effects of either binding segment mutations and modifications, or cytokine mutations and modifications, e.g., IL-D80 analogs.

The use of competitive drug screening assays is contemplated, e.g., where neutralizing antibodies to the cytokine, or receptor binding fragments compete with a test compound.

"Derivatives" of IL-D80 antigens include amino acid sequence mutants from naturally occurring forms, glycosylation variants, and covalent or aggregate conjugates with other chemical moieties. Covalent derivatives can be prepared by linkage of functionalities to groups which are found in IL-D80 amino acid side chains or at the N- or C-termini, e.g., by standard means. See, e.g., Lundblad and Noyes (1988) Chemical Reagents for Protein Modification, vols. 1-2, CRC Press, Inc., Boca Raton, FL; Hugli (ed. 1989) Techniques in Protein Chemistry, Academic Press, San Diego, CA; and Wong (1991) Chemistry of Protein Conjugation and Cross Linking, CRC Press, Boca Raton, FL.

In particular, glycosylation alterations are included, e.g., made by modifying the glycosylation patterns of a polypeptide during its synthesis and processing, or in further processing steps. See, e.g., Elbein (1987) Ann. Rev. Biochem. 56:497-534. Also embraced are versions of the peptides with the same primary amino acid sequence which have other minor modifications, including phosphorylated amino acid residues, e.g., phosphotyrosine, phosphoserine, or phosphothreonine.

Fusion polypeptides between IL-D80s and other homologous or heterologous proteins are also disclosed. Many cytokine receptors or other surface proteins are multimeric, e.g., homodimeric entities, and a repeat construct may have various advantages, including lessened susceptibility to proteolytic cleavage. Typical examples are fusions of a reporter polypeptide, e.g., luciferase, with a segment or domain of a protein, e.g., a receptor-binding segment, so that the presence or location of the fused ligand may be easily determined. See, e.g., Dull, et al., U.S. Patent No. 4,859,609. Other gene fusion partners include bacterial β-galactosidase, trpE, Protein A, β-lactamase, alpha amylase, alcohol dehydrogenase, yeast alpha mating factor, and detection or purification tags such as a FLAG sequence of His6 sequence. See, e.g., Godowski, et al. (1988) Science 241:812-816.

Fusion peptides will typically be made by either recombinant nucleic acid methods or by synthetic polypeptide methods. Techniques for nucleic acid manipulation and expression are described generally, e.g., in Sambrook, et al. (1989) Molecular Cloning: A Laboratory Manual (2d ed.), vols. 1-3, Cold Spring Harbor Laboratory; and Ausubel, et 25 al. (eds. 1993) Current Protocols in Molecular Biology, Greene and Wiley, NY. Techniques for synthesis of polypeptides are described, e.g., in Merrifield (1963) J. Amer. Chem. Soc. 85:2149-2156; Merrifield (1986) Science 232: 341-347; Atherton, et al. (1989) Solid Phase Peptide Synthesis: A Practical Approach, IRL Press, Oxford; and Grant (1992) Synthetic Peptides: A User's Guide, W.H. Freeman, NY. Refolding methods may be applicable to synthetic proteins.

The use of derivatives of IL-D80 proteins other than variations in amino acid sequence or glycosylation is contemplated. Such derivatives may involve covalent or aggregative association with chemical moieties or protein carriers. Covalent or aggregative derivatives will be useful as immunogens, as reagents in immunoassays, or in purification methods such as for affinity purification of binding partners, e.g., other antigens. An IL-D80 can be immobilized by covalent bonding to a solid support such as cyanogen bromide-activated SEPHAROSE, by methods which are well known in the art, or adsorbed onto polyolefin surfaces, with or without glutaraldehyde cross-linking, for use in the assay or purification of anti-IL-D80 antibodies or an alternative binding composition. The IL-D80 proteins can also be labeled with a detectable group, e.g., for use in diagnostic assays. Purification of IL-D80 may be effected by an immobilized antibody or complementary binding partner, e.g., binding portion of a receptor.

A solubilized IL-D80 or fragment can be used as an immunogen for the production of antisera or antibodies specific for binding. Purified antigen can be used to screen monoclonal antibodies or antigen-binding fragments, encompassing antigen binding fragments of natural antibodies, e.g., Fab, Fab', F(ab)2, etc. Purified IL-D80 antigens can also be used as a reagent to detect antibodies generated in response to the presence of elevated levels of the cytokine, which may be diagnostic of an abnormal or specific physiological or disease condition. This invention contemplates antibodies raised against amino acid sequences encoded by nucleotide sequence shown in SEQ ID NO: 1, 3, 7, or 9, or fragments of proteins containing it. In particular, this invention contemplates antibodies having binding affinity to or being raised against specific domains, e.g., helices A, B, C, or D.

Dissection of critical structural elements which effect the various physiological functions mediated through these antigens is possible using standard techniques of modern molecular biology, particularly in comparing members of the related class. See, e.g., the homolog-scanning mutagenesis technique described in Cunningham, et al. (1989) Science 243:1339-1336; and approaches used in O'Dowd, et al. (1988) J Biol. Chem. 263:15985-15992; and Lechleiter, et al. (1990) EMBO J. 9:4381-4390.

Intracellular functions would probably involve receptor signaling. However, protein internalization may occur under certain circumstances, and interaction between intracellular components and cytokine may occur. Specific segments of interaction of IL-D80 with interacting components may be identified by mutagenesis or direct biochemical means, e.g., cross-linking or affinity methods. Structural analysis by crystallographic or other physical methods will also be applicable. Further investigation of the mechanism of signal transduction will include study of associated components which may be isolatable by affinity methods or by genetic means, e.g., complementation analysis of mutants. Further study of the expression and control of IL-D80 will be pursued. The controlling elements associated with the antigens should exhibit differential physiological, developmental, tissue specific, or other expression patterns. Upstream or downstream genetic regions, e.g., control elements, are of interest.

Structural studies of the IL-D80 antigens will lead to design of new antigens, particularly analogs exhibiting agonist or antagonist properties on the molecule. This can be combined with previously described screening methods to isolate antigens exhibiting desired spectra of activities.

### V. Antibodies

Antibodies can be raised to various epitopes of the IL-D80 proteins, including species, polymorphic, or allelic variants, and fragments thereof, both in their naturally occurring forms and in their recombinant forms. Additionally, antibodies can be raised to IL-D80s in either their active forms or in their inactive forms, including native or denatured versions. Anti-idiotypic antibodies are also contemplated.

Antibodies, including binding fragments and single chain versions, against predetermined fragments of the antigens can be raised by immunization of animals with conjugates of the fragments with immunogenic proteins. Monoclonal antibodies are prepared from cells secreting the desired antibody. These antibodies can be screened for binding to normal or defective IL-D80s, or screened for agonistic or antagonistic activity, e.g., mediated through a receptor. Antibodies may be agonistic or antagonistic, e.g., by sterically blocking binding to a receptor. These monoclonal antibodies will usually bind with at least a K_{D} of about 1 mM, more usually at least about 300 µM, typically at least about 100 µM, more typically at least about 30 µM, preferably at least about 10 µM, and more preferably at least about 3 µM or better.

An IL-D80 protein that specifically binds to or that is specifically immunoreactive with an antibody generated against a defined immunogen, such as an immunogen consisting of the amino acid sequence of SEQ ID NO: 2, 4, 8, or 10, is typically determined in an immunoassay. The immunoassay typically uses a polyclonal antiserum which was raised, e.g., to a polypeptide of SEQ ID NO: 2, 4, 8, or 10. This antiserum is selected to have low crossreactivity against other IL-11, e.g., human or rodent IL-11, preferably from the same species, and any such crossreactivity is removed by immunoabsorption prior to use in the immunoassay.

In order to produce antisera for use in an immunoassay, the protein of SEQ ID NO: 2, 4, 8, or 10, or a combination thereof, is isolated as described herein. For example, recombinant protein may be produced in a mammalian cell line. An appropriate host, e.g., an inbred strain of mice such as Balb/c, is immunized with the selected protein, typically using a standard adjuvant, such as Freund's adjuvant, and a standard mouse immunization protocol (see Harlow and Lane, supra). Alternatively, a synthetic peptide derived from the sequences disclosed herein and conjugated to a carrier protein can be used an immunogen. Polyclonal sera are collected and titered against the immunogen protein in an immunoassay, e.g., a solid phase immunoassay with the immunogen immobilized on a solid support. Polyclonal antisera with a titer of 10⁴ or greater are selected and tested for their cross reactivity against other IL-11 family members, e.g., rodent IL-11, using a competitive binding immunoassay such as the one described in Harlow and Lane, supra, at pages 570-573. Preferably at least one other IL-11 family member is used in this determination in conjunction with, e.g., the primate IL-11. The IL-11 family members can be produced as recombinant proteins and isolated using standard molecular biology and protein chemistry techniques as described herein.

Immunoassays in the competitive binding format can be used for the crossreactivity determinations. For example, the protein of SEQ ID NO: 2 or 8 can be immobilized to a solid support. Proteins added to the assay compete with the binding of the antisera to the immobilized antigen. The ability of the above proteins to compete with the binding of the antisera to the immobilized protein is compared to the protein of SEQ ID NO: 2 or 8. The percent crossreactivity for the above proteins is calculated, using standard calculations. Those antisera with less than 10% crossreactivity with each of the proteins listed above are selected and pooled. The cross-reacting antibodies are then removed from the pooled antisera by immunoabsorption with the above-listed proteins.

The immunoabsorbed and pooled antisera are then used in a competitive binding immunoassay as described above to compare a second protein to the immunogen protein (e.g., the IL-11 like protein of SEQ ID NO: 2, 4, 8, or 10). In order to make this comparison, the two proteins are each assayed at a wide range of concentrations and the amount of each protein required to inhibit 50% of the binding of the antisera to the immobilized protein is determined. If the amount of the second protein required is less than twice the amount of the protein of the selected protein or proteins that is required, then the second protein is said to specifically bind to an antibody generated to the immunogen.

The antibodies of this invention can also be useful in diagnostic applications. As capture or non-neutralizing antibodies, they can be screened for ability to bind to the antigens without inhibiting binding to a receptor. As neutralizing antibodies, they can be useful in competitive binding assays. They will also be useful in detecting or quantifying IL-D80 protein or its receptors. See, e.g., Chan (ed. 1987) Immunology: A Practical Guide, Academic Press, Orlando, FL; Price and Newman (eds. 1991) Principles and Practice of Immunoassay, Stockton Press, N.Y.; and Ngo (ed. 1988) Nonisotopic Immunoassay, Plenum Press, N.Y. Cross absorptions, depletions, or other means will provide preparations of defined selectivity, e.g., unique or shared species specificities. These may be the basis for tests which will identify various groups of antigens.

Further, the antibodies, including antigen binding fragments, of this invention can be potent antagonists that bind to the antigen and inhibit functional binding, e.g., to a receptor which may elicit a biological response. They also can be useful as non-neutralizing antibodies and can be coupled to toxins or radionuclides so that when the antibody binds to antigen, a cell expressing it, e.g., on its surface, is killed. Further, these antibodies can be conjugated to drugs or other therapeutic agents, either directly or indirectly by means of a linker, and may effect drug targeting.

Antigen fragments may be joined to other materials, particularly polypeptides, as fused or covalently joined polypeptides to be used as immunogens. An antigen and its fragments may be fused or covalently linked to a variety of immunogens, such as keyhole limpet hemocyanin, bovine serum albumin, tetanus toxoid, etc. See Microbiology, Hoeber Medical Division, Harper and Row, 1969; Landsteiner (1962) Specificity of Serological Reactions, Dover Publications, New York; Williams, et al. (1967) Methods in Immunology and Immunochemistry, vol. 1, Academic Press, New York; and Harlow and Lane (1988) Antibodies: A Laboratory Manual, CSH Press, NY, for descriptions of methods of preparing polyclonal antisera.

In some instances, it is desirable to prepare monoclonal antibodies from various mammalian hosts, such as mice, rodents, primates, humans, etc. Description of techniques for preparing such monoclonal antibodies may be found in, e.g., Stites, et al. (eds.) Basic and Clinical Immunology (4th ed.), Lange Medical Publications, Los Altos, CA, and references cited therein; Harlow and Lane (1988) Antibodies: A Laboratory Manual, CSH Press; Goding (1986) Monoclonal Antibodies: Principles and Practice (2d ed.), Academic Press, New York; and particularly in Kohler and Milstein (1975) in Nature 256:495-497, which discusses one method of generating monoclonal antibodies.

Other suitable techniques involve in vitro exposure of lymphocytes to the antigenic polypeptides or alternatively to selection of libraries of antibodies in phage or similar vectors. See, Huse, et al. (1989) "Generation of a Large Combinatorial Library of the Immunoglobulin Repertoire in Phage Lambda," Science 246:1275-1281; and Ward, et al. (1989) Nature 341:544-546. The polypeptides and antibodies of the present invention may be used with or without modification, including chimeric or humanized antibodies. Frequently, the polypeptides and antibodies will be labeled by joining, either covalently or non-covalently, a substance which provides for a detectable signal. A wide variety of labels and conjugation techniques are known and are reported extensively in both the scientific and patent literature. Suitable labels include radionuclides, enzymes, substrates, cofactors, inhibitors, fluorescent moieties, chemiluminescent moieties, magnetic particles, and the like. Patents, teaching the use of such labels include U.S. Patent Nos. 3,817,837; 3,850,752; 3,939,350; 3,996,345; 4,277,437; 4,275,149; and 4,366,241. Also, recombinant immunoglobulins may be produced, see Cabilly, U.S. Patent No. 4,816,567; Moore, et al., U.S. Patent No. 4,642,334; and Queen, et al. (1989) Proc. Nat'l Acad. Sci. USA 86:10029-10033.

The antibodies of this invention can also be used for affinity chromatography in isolating the protein. Columns can be prepared where the antibodies are linked to a solid support. See, e.g., Wilchek et al. (1984) Meth. Enzymol. 104:3-55. The converse may be used to purify antibodies.

Antibodies raised against each IL-D80 will also be useful to raise anti-idiotypic antibodies. These will be useful in detecting or diagnosing various immunological conditions related to expression of the respective antigens.

### VI. Nucleic Acids

The described peptide sequences and the related reagents are useful in detecting, isolating, or identifying a DNA clone encoding IL-D80, e.g., from a natural source. Typically, it will be useful in isolating a gene from mammal, and similar procedures will be applied to isolate genes from other species, e.g., warm blooded animals, such as birds and mammals. Cross hybridization will allow isolation of IL-D80 from the same, e.g., polymorphic variants, or other species. A number of different approaches will be available to successfully isolate a suitable nucleic acid clone.

The purified protein or defined peptides are useful for generating antibodies by standard methods, as described above. Synthetic peptides or purified protein can be presented to an immune system to generate monoclonal or polyclonal antibodies. See, e.g., Coligan (1991) Current Protocols in Immunology Wiley/Greene; and Harlow and Lane (1989) Antibodies: A Laboratory Manual, Cold Spring Harbor Press.

For example, the specific binding composition could be used for screening of an expression library made from a cell line which expresses an IL-D80. Screening of intracellular expression can be performed by various staining or immunofluorescence procedures. Binding compositions could be used to affinity purify or sort out cells expressing a surface fusion protein.

The peptide segments can also be used to predict appropriate oligonucleotides to screen a library. The genetic code can be used to select appropriate oligonucleotides useful as probes for screening. See, e.g., SEQ ID NO: 1, 3, 7, or 9. In combination with polymerase chain reaction (PCR) techniques, synthetic oligonucleotides will be useful in selecting correct clones from a library. Complementary sequences will also be used as probes, primers, or antisense strands. Various fragments should be particularly useful, e.g., coupled with anchored vector or poly-A complementary PCR techniques or with complementary DNA of other peptides.

Isolated DNA or fragments may be used to encode an antigenic or biologically active corresponding IL-D80 polypeptide, particularly lacking the portion coding the untranslated 5' portion of the described sequence. In addition, isolated or recombinant DNA is described which encodes a biologically active protein or polypeptide and which is capable of hybridizing under appropriate conditions with the DNA sequences described herein. Said biologically active protein or polypeptide can be an intact antigen, or fragment, and have an amino acid sequence disclosed in, e.g., SEQ ID NO: 2, 4, 8, or 10, particularly a mature, secreted polypeptide. The use of isolated or recombinant DNA, or fragments thereof, which encode proteins which exhibit high identity to a secreted IL-D80 is described. The isolated DNA can have the respective regulatory sequences in the 5' and 3' flanks, e.g., promoters, enhancers, poly-A addition signals, and others. Alternatively, expression may be effected by operably linking a coding segment to a heterologous promoter, e.g., by inserting a promoter upstream from an endogenous gene.

An "isolated" nucleic acid is a nucleic acid, e.g., an RNA, DNA, or a mixed polymer, which is substantially separated from other components which naturally accompany a native sequence, e.g., ribosomes, polymerases, and/or flanking genomic sequences from the originating species. The term embraces a nucleic acid sequence which has been removed from its naturally occurring environment, and includes recombinant or cloned DNA isolates and chemically synthesized analogs or analogs biologically synthesized by heterologous systems. A substantially pure molecule includes isolated forms of the molecule. Generally, the nucleic acid will be in a vector or fragment less than about 50 kb, usually less than about 30 kb, typically less than about 10 kb, and preferably less than about 6 kb.

An isolated nucleic acid will generally be a homogeneous composition of molecules, but will, in some embodiments, contain minor heterogeneity. This heterogeneity is typically found at the polymer ends or portions not critical to a desired biological function or activity.

A "recombinant" nucleic acid is defined either by its method of production or its structure. In reference to its method of production, e.g., a product made by a process, the process is use of recombinant nucleic acid techniques, e.g., involving human intervention in the nucleotide sequence, typically selection or production. Alternatively, it can be a nucleic acid made by generating a sequence comprising fusion of two fragments which are not naturally contiguous to each other, but is meant to exclude products of nature, e.g., naturally occurring mutants. Thus, e.g., products made by transforming cells with any unnaturally occurring vector is encompassed, as are nucleic acids comprising sequence derived using any synthetic oligonucleotide process. Such is often done to replace a codon with a redundant codon encoding the same or a conservative amino acid, while typically introducing or removing a sequence recognition site.

Alternatively, it is performed to join together nucleic acid segments of desired functions to generate a single genetic entity comprising a desired combination of functions not found in the commonly available natural forms. Restriction enzyme recognition sites are often the target of such artificial manipulations, but other site specific targets, e.g., promoters, DNA replication sites, regulation sequences, control sequences, or other useful features may be incorporated by design. A similar concept is intended for a recombinant, e.g., fusion, polypeptide. Specifically included are synthetic nucleic acids which, by genetic code redundancy, encode polypeptides similar to fragments of these antigens, and fusions of sequences from various different species or polymorphic variants.

A significant "fragment" in a nucleic acid context is a contiguous segment of at least about 17 nucleotides, generally at least about 22 nucleotides, ordinarily at least about 29 nucleotides, more often at least about 35 nucleotides, typically at least about 41 nucleotides, usually at least about 47 nucleotides, preferably at least about 55 nucleotides, and in particularly preferred embodiments will be at least about 60 or more nucleotides, e.g., 67, 73, 81, 89, 95, etc.

A DNA which codes for an IL-D80 protein will be particularly useful to identify genes, mRNA, and cDNA species which code for related or similar proteins, as well as DNAs which code for homologous proteins from different species. There will be homologs in other species, including primates, rodents, canines, felines, birds, and fish. Various IL-D80 proteins should be homologous and are encompassed herein. However, even proteins that have a more distant evolutionary relationship to the antigen can readily be isolated under appropriate conditions using these sequences if they are sufficiently homologous. Primate IL-D80 proteins are of particular interest.

Recombinant clones derived from the genomic sequences, e.g., containing introns, will be useful for transgenic studies, including, e.g., transgenic cells and organisms, and for gene therapy. See, e.g., Goodnow (1992) "Transgenic Animals" in Roitt (ed.) Encyclopedia of Immunology, Academic Press, San Diego, pp. 1502-1504; Travis (1992) Science 256:1392-1394; Kuhn, et al. (1991) Science 254:707-710; Capecchi (1989) Science 244:1288; Robertson (ed. 1987) Teratocarcinomas and Embryonic Stem Cells: A Practical Approach, IRL Press, Oxford; Rosenberg (1992) J. Clinical Oncology 10:180-199; and Cournoyer and Caskey (1993) Ann. Rev. Immunol. 11:297-329. Alternatively, expression may be effected by operably linking a coding segment to a heterologous promoter, e.g., by inserting a promoter upstream from an endogenous gene. See, e.g., Treco, et al. WO96/29411 or USSN 08/406,030.

Substantial homology, e.g., identity, in the nucleic acid sequence comparison context means either that the segments, or their complementary strands, when compared, are identical when optimally aligned, with appropriate nucleotide insertions or deletions, in at least about 50% of the nucleotides, generally at least about 58%, ordinarily at least about 65%, often at least about 71%, typically at least about 77%, usually at least about 85%, preferably at least about 95 to 98% or more, and in particular embodiments, as high as about 99% or more of the nucleotides. Alternatively, substantial homology exists when the segments will hybridize under selective hybridization conditions, to a strand, or its complement, typically using a sequence of IL-D80, e.g., in SEQ ID NO: 1, 3, 7, or 9. Typically, selective hybridization will occur when there is at least about 55% identity over a stretch of at least about 30 nucleotides, preferably at least about 75% over a stretch of about 25 nucleotides, and most preferably at least about 90% over about 20 nucleotides. See, Kanehisa (1984) Nuc. Acids Res. 12:203-213. The length of identity comparison, as described, may be over longer stretches, and in certain embodiments will be over a stretch of at least about 17 nucleotides, usually at least about 28 nucleotides, typically at least about 40 nucleotides, and preferably at least about 75 to 100 or more nucleotides.

Stringent conditions, in referring to homology in the hybridization context, will be stringent combined conditions of salt, temperature, organic solvents, and other parameters, typically those controlled in hybridization reactions. Stringent temperature conditions will usually include temperatures in excess of about 30° C, usually in excess of about 37° C, typically in excess of about 55° C, 60° C, or 65° C, and preferably in excess of about 70° C. Stringent salt conditions will ordinarily be less than about 1000 mM, usually less than about 400 mM, typically less than about 250 mM, preferably less than about 150 mM, including about 100, 50, or even 20 mM. However, the combination of parameters is much more important than the measure of any single parameter. See, e.g., Wetmur and Davidson (1968) J. Mol. Biol. 31:349-370. Hybridization under stringent conditions should give a background of at least 2-fold over background, preferably at least 3-5 or more.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optical alignment of sequences for comparison can be conducted, e.g., by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Nat'l Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel et al., supra).

One example of a useful algorithm is PILEUP. PILEUP creates a multiple sequence alignment from a group of related sequences using progressive, pairwise alignments to show relationship and percent sequence identity. It also plots a tree or dendrogram showing the clustering relationships used to create the alignment. PILEUP uses a simplification of the progressive alignment method of Feng and Doolittle (1987) J. Mol. Evol. 35:351-360. The method used is similar to the method described by Higgins and Sharp (1989) CABIOS 5:151-153. The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters. For example, a reference sequence can be compared to other test sequences to determine the percent sequence identity relationship using the following parameters: default gap weight (3.00), default gap length weight (0.10), and weighted end gaps.

Another example of algorithm that is suitable for determining percent sequence identity and sequence similarity is the BLAST algorithm, which is described Altschul, et al. (1990) J. Mol. Biol. 215:403-410. Software for performing BLAST analyses is publicly available through the National Center for Biotechnology Information (http:www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul, et al., supra). These initial neighborhood word hits act as seeds for initiating searches to find longer HSPs containing them. The word hits are then extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff and Henikoff (1989) Proc. Nat'l Acad. Sci. USA 89:10915) alignments (B) of 50, expectation (E) of 10, M=5, N=4, and a comparison of both strands.

In addition to calculating percent sequence identity, the BLAST algorithm also performs a statistical analysis of the similarity between two sequences (see, e.g., Karlin and Altschul (1993) Proc. Nat'l Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

A further indication that two nucleic acid sequences of polypeptides are substantially identical is that the polypeptide encoded by the first nucleic acid is immunologically cross reactive with the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules hybridize to each other under stringent conditions, as described below.

IL-D80 from other mammalian species can be cloned and isolated by cross-species hybridization of closely related species. Homology may be relatively low between distantly related species, and thus hybridization of relatively closely related species is advisable. Alternatively, preparation of an antibody preparation which exhibits less species specificity may be useful in expression cloning approaches.

### VII. Making IL-D80; Mimetics

DNA which encodes the IL-D80 or fragments thereof can be obtained by chemical synthesis, screening cDNA libraries, or screening genomic libraries prepared from a wide variety of cell lines or tissue samples. See, e.g., Okayama and Berg (1982) Mol. Cell. Biol. 2:161-170; Gubler and Hoffman (1983) Gene 25:263-269; and Glover (ed. 1984) DNA Cloning: A Practical Approach, IRL Press, Oxford. Alternatively, the sequences provided herein provide useful PCR primers or allow synthetic or other preparation of suitable genes encoding an IL-D80; including naturally occurring embodiments.

This DNA can be expressed in a wide variety of host cells for the synthesis of a full-length IL-D80 or fragments which can in turn, e.g., be used to generate polyclonal or monoclonal antibodies; for binding studies; for construction and expression of modified molecules; and for structure/function studies. There may be a need for a chaparone protein for efficient secretion, or additional steps may be necessary to retrieve the protein from the intracellular compartment.

Vectors, as used herein, comprise plasmids, viruses, bacteriophage, integratable DNA fragments, and other vehicles which enable the integration of DNA fragments into the genome of the host. See, e.g., Pouwels, et al. (1985 and Supplements) Cloning Vectors: A Laboratory Manual, Elsevier, N.Y.; and Rodriguez, et al. (eds. 1988) Vectors: A Survey of Molecular Cloning Vectors and Their Uses, Buttersworth, Boston, MA.

For purposes of this invention, DNA sequences are operably linked when they are functionally related to each other. For example, DNA for a presequence or secretory leader is operably linked to a polypeptide if it is expressed as a preprotein or participates in directing the polypeptide to the cell membrane or in secretion of the polypeptide. A promoter is operably linked to a coding sequence if it controls the transcription of the polypeptide; a ribosome binding site is operably linked to a coding sequence if it is positioned to permit translation. Usually, operably linked means contiguous and in reading frame, however, certain genetic elements such as repressor genes are not contiguously linked but still bind to operator sequences that in turn control expression. See, e.g., Rodriguez, et al., Chapter 10, pp. 205-236; Balbas and Bolivar (1990) Methods in Enzymology 185:14-37; and Ausubel, et al. (1993) Current Protocols in Molecular Biology, Greene and Wiley, NY.

Representative examples of suitable expression vectors include pCDNA1; pCD, see Okayama, et al. (1985) Mol. Cell Biol. 5:1136-1142; pMClneo Poly-A, see Thomas, et al. (1987) Cell 51:503-512; and a baculovirus vector such as pAC 373 or pAC 610. See, e.g., Miller (1988) Ann. Rev. Microbiol. 42:177-199.

It will often be desired to express an IL-D80 polypeptide in a system which provides a specific or defined glycosylation pattern. See, e.g., Luckow and Summers (1988) Bio/Technoloqv 6:47-55; and Kaufman (1990) Meth. Enzvmol. 185:487-511.

The IL-D80, or a fragment thereof, may be engineered to be phosphatidyl inositol (PI) linked to a cell membrane, but can be removed from membranes by treatment with a phosphatidyl inositol cleaving enzyme, e.g., phosphatidyl inositol phospholipase-C. This releases the antigen in a biologically active form, and allows purification by standard procedures of protein chemistry. See, e.g., Low (1989) Biochim. Biophys. Acta 988:427-454; Tse, et al. (1985) Science 230:1003-1008; and Brunner, et al. (1991) J. Cell Biol. 114:1275-1283.

Now that the IL-D80 has been characterized, fragments or derivatives thereof can be prepared by conventional processes for synthesizing peptides. These include processes such as are described in Stewart and Young (1984) Solid Phase Peptide Synthesis, Pierce Chemical Co., Rockford, IL; Bodanszky and Bodanszky (1984) The Practice of Peptide Synthesis, Springer-Verlag, New York; Bodanszky (1984) The Principles of Peptide Synthesis, Springer-Verlag, New York; and Villafranca (ed. 1991) Techniques in Protein Chemistry II, Academic Press, San Diego, Ca.

### VIII. Uses

The present invention provides reagents which will find use in diagnostic applications as described elsewhere herein, e.g., in IL-D80 mediated conditions, or below in the description of kits for diagnosis.

This invention also provides reagents with significant commercial and/or therapeutic potential. The IL-D80 antibodies should be useful as reagents for teaching techniques of molecular biology, immunology, or physiology. Appropriate kits may be prepared with the reagents, e.g., in practical laboratory exercises in production or use of proteins, antibodies, cloning methods, histology, etc.

The reagents will also be useful in the treatment of conditions associated with abnormal physiology or development, including inflammatory conditions. They may be useful in vitro tests for presence or absence of interacting components, which may correlate with success of particular treatment strategies. In particular, modulation of physiology of various, e.g., hematopoietic or lymphoid, cells will be achieved by appropriate methods for treatment using the compositions provided herein. See, e.g., Thomson (ed. 1998) The Cytokine Handbook (3d ed.) Academic Press, San Diego; Metcalf and Nicola (1995) The Hematopoietic Colony Stimulating Factors Cambridge University Press; and Aggarwal and Gutterman (1991) Human Cytokines Blackwell Pub.

For example, a disease or disorder associated with abnormal expression or abnormal signaling by an IL-D80 should be a likely target for an agonist or antagonist. The new cytokine should play a role in regulation or development of hematopoietic cells, e.g., lymphoid cells, which affect immunological responses, e.g., inflammation and/or autoimmune disorders. Alternatively, it may affect vascular physiology or development, or neuronal effects.

In particular, the cytokine should mediate, in various contexts, cytokine synthesis by the cells, proliferation, etc. Antagonists of IL-D80, such as blocking antibodies, may provide a selective and powerful way to block immune responses, e.g., in situations as inflammatory or autoimmune responses. See also Samter, et al. (eds.) Immunological Diseases vols. 1 and 2, Little, Brown and Co.

Various abnormal conditions are known in different cell types which will produce IL-D80, e.g., as evaluated by mRNA expression by Northern blot analysis. See Berkow (ed.) The Merck Manual of Diagnosis and Therapy, Merck & Co., Rahway, N.J.; Thorn, et al. Harrison's Principles of Internal Medicine, McGraw-Hill, N.Y.; and Weatherall, et al. (eds.) Oxford Textbook of Medicine, Oxford University Press, Oxford. Many other medical conditions and diseases involve activation by macrophages or monocytes, and many of these will be responsive to treatment by an agonist or antagonist provided herein. See, e.g., Stites and Terr (eds.; 1991) Basic and Clinical Immunology Appleton and Lange, Norwalk, Connecticut; and Samter, et al. (eds.) Immunological Diseases Little, Brown and Co. These problems should be susceptible to prevention or treatment using compositions provided herein.

IL-D80 antibodies can be purified and then administered to a patient, veterinary or human. These reagents can be combined for therapeutic use with additional active or inert ingredients, e.g., in conventional pharmaceutically acceptable carriers or diluents, e.g., immunogenic adjuvants, along with physiologically innocuous stabilizers, excipients, or preservatives. These combinations can be sterile filtered and placed into dosage forms as by lyophilization in dosage vials or storage in stabilized aqueous preparations. This invention also contemplates use of antibodies or binding fragments thereof, including forms which are not complement binding.

Drug screening using IL-D80 or fragments thereof can be performed to identify compounds having binding affinity to or other relevant biological effects on IL-D80 functions, including isolation of associated components. Subsequent biological assays can then be utilized to determine if the compound has intrinsic stimulating activity and is therefore a blocker or antagonist in that it blocks the activity of the cytokine. Likewise, a compound having intrinsic stimulating activity can activate the signal pathway and is thus an agonist in that it simulates the activity of IL-D80. This invention further contemplates the therapeutic use of blocking antibodies to IL-D80 as antagonists and of stimulatory antibodies as agonists. This approach should be particularly useful with other IL-D80 species variants.

The quantities of reagents necessary for effective therapy will depend upon many different factors, including means of administration, target site, physiological state of the patient, and other medicants administered. Thus, treatment dosages should be titrated to optimize safety and efficacy. Typically, dosages used in vitro may provide useful guidance in the amounts useful for in situ administration of these reagents. Animal testing of effective doses for treatment of particular disorders will provide further predictive indication of human dosage. Various considerations are described, e.g., in Gilman, et al. (eds.) Goodman and Gilman's: The Pharmacological Bases of Therapeutics, latest Ed., Pergamon Press; and Remington's Pharmaceutical Sciences, latest ed., Mack Publishing Co., Easton, Penn. Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, New Jersey. Dosage ranges would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier. Slow release formulations, or a slow release apparatus will often be utilized for continuous or long term administration. See, e.g., Langer (1990) Science 249:1527-1533.

IL-D80 antibodies may be administered directly to the host to be treated or, depending on the size of the compounds, it may be desirable to conjugate them to carrier proteins such as ovalbumin or serum albumin prior to their administration. Therapeutic formulations may be administered in many conventional dosage formulations. While it is possible for the active ingredient to be administered alone, it is preferable to present it as a pharmaceutical formulation. Formulations typically comprise at least one active ingredient, as defined above, together with one or more acceptable carriers thereof. Each carrier should be both pharmaceutically and physiologically acceptable in the sense of being compatible with the other ingredients and not injurious to the patient. Formulations include those suitable for oral, rectal, nasal, topical, or parenteral (including subcutaneous, intramuscular, intravenous and intradermal) administration. The formulations may conveniently be presented in unit dosage form and may be prepared by any methods well known in the art of pharmacy. See, e.g., Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Penn.; Avis, et al. (eds. 1993) Pharmaceutical Dosage Forms: Parenteral Medications, Dekker, New York; Lieberman, et al. (eds. 1990) Pharmaceutical Dosage Forms: Tablets, Dekker, New York; and Lieberman, et al. (eds. 1990) Pharmaceutical Dosage Forms: Disperse Systems, Dekker, New York. The therapy of this invention may be combined with or used in association with other agents, e.g., other cytokines, including IL-11, or its antagonists.

Both naturally occurring and recombinant forms of the IL-D80s are particularly useful in kits and assay methods which are capable of screening compounds for binding activity to the proteins. Several methods of automating assays have been developed in recent years so as to permit screening of tens of thousands of compounds in a short period. See, e.g., Fodor, et al. (1991) Science 251:767-773, which describes means for testing of binding affinity by a plurality of defined polymers synthesized on a solid substrate. The development of suitable assays can be greatly facilitated by the availability of large amounts of purified, soluble IL-D80.

Other methods can be used to determine the critical residues in IL-080-IL-D80 receptor interactions. Mutational analysis can be performed, e.g., see Somoza, et al. (1993) J. Exptl. Med. 178:549-558, to determine specific residues critical in the interaction and/or signaling. PHD (Rost and Sander (1994) Proteins 19:55-72) and DSC (King and Sternberg (1996) Protein Sci. 5:2298-2310) can provide secondary structure predictions of α-helix (H), β-stand (E), or coil (L). Helices A and D are most important in receptor interaction, with the D helix the more important region. Boundaries for the various helices are indicated above. Surface exposed residues would affect receptor binding, while embedded residues would affect general structure.

For example, antagonists can normally be found once the antigen has been structurally defined, e.g., by tertiary structure data. Testing of potential interacting analogs is now possible upon the development of highly automated assay methods using a purified IL-D80. In particular, new agonists and antagonists will be discovered by using screening techniques described herein. Of particular importance are compounds found to have a combined binding affinity for a spectrum of IL-D80 molecules, e.g., compounds which can serve as antagonists for species variants of IL-D80.

One method of drug screening utilizes eukaryotic or prokaryotic host cells which are stably transformed with recombinant DNA molecules expressing an IL-D80. Cells may be isolated which express an IL-D80 in isolation from other molecules. Such cells, either in viable or fixed form, can be used for standard binding partner binding assays. See also, Parce, et al. (1989) Science 246:243-247; and Owicki, et al. (1990) Proc. Nat'l Acad. Sci. USA 87:4007-4011, which describe sensitive methods to detect cellular responses.

Another technique for drug screening involves an approach which provides high throughput screening for compounds having suitable binding affinity to an IL-D80 and is described in detail in Geysen, European Patent Application 84/03564, published on September 13, 1984. First, large numbers of different small peptide test compounds are synthesized on a solid substrate, e.g., plastic pins or some other appropriate surface, see Fodor, et al. (1991). Then all the pins are reacted with solubilized, unpurified or solubilized, purified IL-D80, and washed. The next step involves detecting bound IL-D80.

Rational drug design may also be based upon structural studies of the molecular shapes of the IL-D80 and other effectors or analogs. Effectors may be other proteins which mediate other functions in response to binding, or other proteins which normally interact with IL-D80, e.g, a receptor. One means for determining which sites interact with specific other proteins is a physical structure determination, e.g., x-ray crystallography or 2 dimensional NMR techniques. These will provide guidance as to which amino acid residues form molecular contact regions, as modeled, e.g., against other cytokine-receptor models. For a detailed description of protein structural determination, see, e.g., Blundell and Johnson (1976) Protein Crystallography, Academic Press, New York.

### IX. Kits

This invention also contemplates a variety of diagnostic kits and methods for detecting the presence of another IL-D80 or binding partner. Typically the kit will have a compartment containing either a defined IL-D80 peptide or IL-D80 antibodies.

A kit for determining the binding affinity of a test compound to an IL-D80 would typically comprise a test compound; a labeled compound, for example a binding antibody having known binding affinity for IL-D80; a source of IL-D80 (naturally occurring or recombinant); and a means for separating bound from free labeled compound, such as a solid phase for immobilizing the molecule. Once compounds are screened, those having suitable binding affinity to the antigen can be evaluated in suitable biological assays, as are well known in the art, to determine whether they act as agonists or antagonists to the IL-D80 signaling pathway. The availability of recombinant IL-D80 polypeptides also provide well defined standards for calibrating such assays.

A preferred kit for determining the concentration of, e.g., an IL-D80 in a sample would typically comprise a labeled compound, e.g., binding partner or antibody, having known binding affinity for the antigen, a source of cytokine (naturally occurring or recombinant) and a means for separating the bound from free labeled compound, e.g., a solid phase for immobilizing the IL-D80. Compartments containing reagents, and instructions, will normally be provided.

Antibodies, including antigen binding fragments, specific for the IL-D80 or fragments are useful in diagnostic applications to detect the presence of elevated levels of IL-D80 and/or its fragments. Such diagnostic assays can employ lysates, live cells, fixed cells, immunofluorescence, cell cultures, body fluids, and further can involve the detection of antigens related to the antigen in serum, or the like. Diagnostic assays may be homogeneous (without a separation step between free reagent and antigen-binding partner complex) or heterogeneous (with a separation step). Various commercial assays exist, such as radioimmunoassay (RIA), enzyme-linked immunosorbent assay (ELISA), enzyme immunoassay (EIA), enzyme-multiplied immunoassay technique (EMIT), substrate-labeled fluorescent immunoassay (SLFIA), and the like. See, e.g., Van Vunakis, et al. (1980) Meth Enzymol. 70:1-525; Harlow and Lane (1980) Antibodies: A Laboratory Manual, CSH Press, NY; and Coligan, et al. (eds. 1993) Current Protocols in Immunology, Greene and Wiley, NY.

Anti-idiotypic antibodies may have similar use to diagnose presence of antibodies against an IL-D80, as such may be diagnostic of various abnormal states. For example, overproduction of IL-D80 may result in production of various immunological reactions which may be diagnostic of abnormal physiological states, particularly in proliferative cell conditions such as cancer or abnormal activation or differentiation. Moreover, the distribution pattern available provides information that the cytokine is expressed in pancreatic islets, suggesting the possibility that the cytokine may be involved in function of that organ, e.g., in a diabetes relevant medical condition.

Frequently, the reagents for diagnostic assays are supplied in kits, so as to optimize the sensitivity of the assay. Depending upon the nature of the assay, the protocol, and the label, either labeled or unlabeled antibody or binding partner, or labeled IL-D80 is used. This is usually in conjunction with other additives, such as buffers, stabilizers, materials necessary for signal production such as substrates for enzymes, and the like. Preferably, the kit will also contain instructions for proper use and disposal of the contents after use. Typically the kit has compartments for each useful reagent. Desirably, the reagents are provided as a dry lyophilized powder, where the reagents may be reconstituted in an aqueous medium providing appropriate concentrations of reagents for performing the assay.

Many of the aforementioned constituents of the drug screening and the diagnostic assays may be used without modification or may be modified in a variety of ways. For example, labeling may be achieved by covalently or non-covalently joining a moiety which directly or indirectly provides a detectable signal. In any of these assays, the binding partner, test compound, IL-D80, or antibodies thereto can be labeled either directly or indirectly. Possibilities for direct labeling include label groups: radiolabels such as ¹²⁵I, enzymes (U.S. Pat. No. 3,645,090) such as peroxidase and alkaline phosphatase, and fluorescent labels (U.S. Pat. No. 3,940,475) capable of monitoring the change in fluorescence intensity, wavelength shift, or fluorescence polarization. Possibilities for indirect labeling include biotinylation of one constituent followed by binding to avidin coupled to one of the above label groups.

There are also numerous methods of separating the bound from the free IL-D80, or alternatively the bound from the free test compound. The IL-D80 can be immobilized on various matrixes followed by washing. Suitable matrixes include plastic such as an ELISA plate, filters, and beads. See, e.g., Coligan, et al. (eds. 1993) Current Protocols in Immunology, Vol. 1, Chapter 2, Greene and Wiley, NY. Other suitable separation techniques include, without limitation, the fluorescein antibody magnetizable particle method described in Rattle, et al. (1984) Clin. Chem. 30:1457-1461, and the double antibody magnetic particle separation as described in U.S. Pat. No. 4,659,678.

Methods for linking proteins or their fragments to the various labels have been extensively reported in the literature and do not require detailed discussion here.

Many of the techniques involve the use of activated carboxyl groups either through the use of carbodiimide or active esters to form peptide bonds, the formation of thioethers by reaction of a mercapto group with an activated halogen such as chloroacetyl, or an activated olefin such as maleimide, for linkage, or the like. Fusion proteins will also find use in these applications.

### X. Isolating an IL-D80 Receptor

Having isolated a ligand of a specific ligand-receptor interaction, methods exist for isolating the receptor. See, Gearing, et al. (1989) EMBO J. 8:3667-3676. For example, means to label the IL-D80 cytokine without interfering with the binding to its receptor can be determined. For example, an affinity label can be fused to either the amino- or carboxyl-terminus of the ligand. Such label may be a FLAG epitope tag, or, e.g., an Ig or Fc domain. An expression library can be screened for specific binding of the cytokine, e.g., by cell sorting, or other screening to detect subpopulations which express such a binding component. See, e.g., Ho, et al. (1993) Proc. Nat'l Acad. Sci. USA 90:11267-11271; and Liu, et al. (1994) J. Immunol. 152:1821-29. Alternatively, a panning method may be used. See e.g., Seed and Aruffo (1987) Proc. Nat'l Acad. Sci. USA 84:3365-3369.

Protein cross-linking techniques with label can be applied to isolate binding partners of the IL-D80 cytokine. This would allow identification of proteins which specifically interact with the cytokine, e.g., in a ligand-receptor like manner. It is a prediction that the IL-D80 will bind to the IL-11R alpha subunit, or a closely homologous receptor subunit. The beta subunit of the receptor is likely to be the gp130, possibly with involvement of the LIF receptor as an additional receptor component.

Early experiments will be performed, as predicted, to determine whether the known IL-11 receptor components are involved in response(s) to IL-D80. It is also quite possible that these functional receptor complexes may share many or all components with an IL-D80 receptor complex, either a specific receptor subunit or an accessory receptor subunit.

### EXAMPLES

### I. General Methods

Many of the standard methods below are described or referenced, e.g., in Maniatis, et al. (1982) Molecular Cloning, A Laboratory Manual Cold Spring Harbor Laboratory, Cold Spring Harbor Press, NY; Sambrook, et al. (1989) Molecular Cloning: A Laboratory Manual (2d ed.) Vols. 1-3, CSH Press, NY; Ausubel, et al., Biology Greene Publishing Associates, Brooklyn, NY; or Ausubel, et al. (1987 and Supplements) Current Protocols in Molecular Biology Wiley/Greene, NY; Innis, et al. (eds. 1990) PCR Protocols: A Guide to Methods and Applications Academic Press, NY. Methods for protein purification include such methods as ammonium sulfate precipitation, column chromatography, electrophoresis, centrifugation, crystallization, and others. See, e.g., Ausubel, et al. (1987 and periodic supplements); Deutscher (1990) "Guide to Protein Purification," Methods in Enzymology vol. 182, and other volumes in this series; Coligan, et al. (1995 and supplements) Current Protocols in Protein Science John Wiley and Sons, New York, NY; P. Matsudaira (ed. 1993) A Practical Guide to Protein and Peptide Purification for Microsequencing, Academic Press, San Diego, CA; and manufacturer's literature on use of protein purification products, e.g., Pharmacia, Piscataway, NJ, or Bio-Rad, Richmond, CA. Combination with recombinant techniques allow fusion to appropriate segments (epitope tags), e.g., to a FLAG sequence or an equivalent which can be fused, e.g., via a protease-removable sequence. See, e.g., Hochuli (1989) Chemische Industrie 12:69-70; Hochuli (1990) "Purification of Recombinant Proteins with Metal Chelate Absorbent" in Setlow (ed.) Genetic Engineering. Principle and Methods 12:87-98, Plenum Press, NY; and Crowe, et al. (1992) OIAexpress: The High Level Expression & Protein Purification System QUIAGEN, Inc., Chatsworth, CA.

Standard immunological techniques are described, e.g., in Hertzenberg, et al. (eds. 1996) Weir's Handbook of Experimental Immunology vols. 1-4, Blackwell Science; Coligan (1991) Current Protocols in Immunology Wiley/Greene, NY; and Methods in Enzymology vols. 70, 73, 74, 84, 92, 93, 108, 116, 121, 132, 150, 162, and 163. Cytokine assays are described, e.g., in Thomson (ed. 1998) The Cytokine Handbook (3d ed.) Academic Press, San Diego; Mire-Sluis and Thorpe (1998) Cytokines Academic Press, San Diego; Metcalf and Nicola (1995) The Hematopoietic Colony Stimulating Factors Cambridge University Press; and Aggarwal and Gutterman (1991) Human Cytokines Blackwell Pub.

Assays for vascular biological activities are well known in the art. They will cover angiogenic and angiostatic activities in tumor, or other tissues, e.g., arterial smooth muscle proliferation (see, e.g., Koyoma, et al. (1996) Cell 87:1069-1078), monocyte adhesion to vascular epithelium (see McEvoy, et al. (1997) J. Exp. Med. 185:2069-2077), etc. See also Ross (1993) Nature 362:801-809; Rekhter and Gordon (1995) Am. J. Pathol. 147:668-677; Thyberg, et al. (1990) Atherosclerosis 10:966-990; and Gumbiner (1996) Cell 84:345-357.

Assays for neural cell biological activities are described, e.g., in Wouterlood (ed. 1995) Neuroscience Protocols modules 10, Elsevier; Methods in Neurosciences Academic Press; and Neuromethods Humana Press, Totowa, NJ. Methodology of developmental systems is described, e.g., in Meisami (ed.) Handbook of Human Growth and Developmental Biology CRC Press; and Chrispeels (ed.) Molecular Techniques and Approaches in Developmental Biology Interscience.

FACS analyses are described in Melamed, et al. (1990) Flow Cytometry and Sorting Wiley-Liss, Inc., New York, NY; Shapiro (1988) Practical Flow Cytometry Liss, New York, NY; and Robinson, et al. (1993) Handbook of Flow Cytometry Methods Wiley-Liss, New York, NY.

### II. Cloning of Human IL-D80

The sequences of primate, e.g., human, genes are provided in Table 1. These sequences are derived from a sequence database. These sequences allow preparation of PCR primers, or probes, to determine cellular distribution of the gene. These sequences allow isolation of genomic DNA which encode the message.

Using the probe or PCR primers, various tissues or cell types are probed to determine cellular distribution. PCR products are cloned using, e.g., a TA cloning kit (Invitrogen). The resulting cDNA plasmids are sequenced from both termini on an automated sequencer (Applied Biosystems).

### III. Cellular Expression of IL-D80

An appropriate probe or primers specific for cDNA encoding primate IL-D80 are prepared. Typically, the probe is labeled, e.g., by random priming.

Southern Analysis: DNA (5 µg) from a primary amplified cDNA library was digested with appropriate restriction enzymes to release the inserts, run on a 1% agarose gel and transferred to a nylon membrane (Schleicher and Schuell, Keene, NH).

Samples for human mRNA isolation may include: peripheral blood mononuclear cells (monocytes, T cells, NK cells, granulocytes, B cells), resting (T100); peripheral blood mononuclear cells, activated with anti-CD3 for 2, 6, 12 h pooled (T101); T cell, TH0 clone Mot 72, resting (T102); T cell, TH0 clone Mot 72, activated with anti-CD28 and anti-CD3 for 3, 6, 12 h pooled (T103); T cell, TH0 clone Mot 72, anergic treated with specific peptide for 2, 7, 12 h pooled (T104); T cell, TH1 clone HY06, resting (T107); T cell, TH1 clone HY06, activated with anti-CD28 and anti-CD3 for 3, 6, 12 h pooled (T108) ; T cell, TH1 clone HY06, anergic treated with specific peptide for 2, 6, 12 h pooled (T109); T cell, TH2 clone HY935, resting (T110); T cell, TH2 clone HY935, activated with anti-CD28 and anti-CD3 for 2, 7, 12 h pooled (T111); T cell tumor lines Jurkat and Hut78, resting (T117); T cell clones, pooled AD130.2, Tc783.12, Tc783.13, Tc783.58, Tc782.69, resting (T118); T cell random γδ T cell clones, resting (T119); CD28- T cell clone; Splenocytes, resting (B100); Splenocytes, activated with anti-CD40 and IL-4 (B101); B cell EBV lines pooled WT49, RSB, JY, CVIR, 721.221, RM3, HSY, resting (B102); B cell line JY, activated with PMA and ionomycin for 1, 6 h pooled (B103); NK 20 clones pooled, resting (K100); NK 20 clones pooled, activated with PMA and ionomycin for 6 h (K101); NKL clone, derived from peripheral blood of LGL leukemia patient, IL-2 treated (K106); hematopoietic precursor line TF1, activated with PMA and ionomycin for 1, 6 h pooled (C100); U937 premonocytic line, resting (M100); U937 premonocytic line, activated with PMA and ionomycin for 1, 6 h pooled (M101); elutriated monocytes, activated with LPS, IFNγ, anti-IL-10 for 1, 2, 6, 12, 24 h pooled (M102); elutriated monocytes, activated with LPS, IFNγ, IL-10 for 1, 2, 6, 12, 24 h pooled (M103); elutriated monocytes, activated with LPS, IFNγ, anti-IL-10 for 4, 16 h pooled (M106); elutriated monocytes, activated with LPS, IFNγ, IL-10 for 4, 16 h pooled (M107); elutriated monocytes, activated LPS for 1 h (M108); elutriated monocytes, activated LPS for 6 h (M109); DC 70% CD1a+, from CD34+ GM-CSF, TNFα 12 days, resting (D101); DC 70% CD1a+, from CD34+ GM-CSF, TNFα 12 days, activated with PMA and ionomycin for 1 hr (D102) ; DC 70% CD1a+, from CD34+ GM-CSF, TNFα 12 days, activated with PMA and ionomycin for 6 hr (D103); DC 95% CD1a+, from CD34+ GM-CSF, TNFα 12 days FACS sorted, activated with PMA and ionomycin for 1, 6 h pooled (D104); DC 95% CD14+, ex CD34+ GM-CSF, TNFα 12 days FACS sorted, activated with PMA and ionomycin 1, 6 hr pooled (D105); DC CD1a+ CD86+, from CD34+ GM-CSF, TNFα 12 days FACS sorted, activated with PMA and ionomycin for 1, 6 h pooled (D106); DC from monocytes GM-CSF, IL-4 5 days, resting (D107); DC from monocytes GM-CSF, IL-4 5 days, resting (D108); DC from monocytes GM-CSF, IL-4 5 days, activated LPS 4, 16 h pooled (D109); DC from monocytes GM-CSF, IL-4 5 days, activated TNFα, monocyte supe for 4, 16 h pooled (D110); epithelial cells, unstimulated; epithelial cells, IL-1β activated; lung fibroblast sarcoma line MRC5, activated with PMA and ionomycin for 1, 6 h pooled (C101); kidney epithelial carcinoma cell line CHA, activated with PMA and ionomycin for 1, 6 h pooled (C102).

A rodent counterpart, e.g., mouse, has been identified, and its distributions will be similarly evaluated. Samples for mouse mRNA isolation can include: resting mouse fibroblastic L cell line (C200); Braf:ER (Braf fusion to estrogen receptor) transfected cells, control (C201); Me114+ naive T cells from spleen, resting (T209); Me114+ naive T cells from spleen, stimulated with IFNγ, IL-12, and anti IL-4 to polarize to TH1 cells, exposed to IFNγ and IL-4 for 6, 12, 24 h, pooled (T210); Me114+ naive T cells from spleen, stimulated with IL-4 and anti IFNγ to polarize to Th2 cells, exposed to IL-4 and anti IFNγ for 6, 13, 24 h, pooled (T211); T cells, TH1 polarized (Me114 bright, CD4+ cells from spleen, polarized for 7 days with IFN-γ and anti IL-4; T200); T cells, TH2 polarized (Me114 bright, CD4+ cells from spleen, polarized for 7 days with IL-4 and anti-IFN-γ; T201); T cells, highly TH1 polarized 3x from transgenic Balb/C (see Openshaw, et al. (1995) J. Exp. Med. 182:1357-1367; activated with anti-CD3 for 2, 6, 24 h pooled; T202); T cells, highly TH2 polarized 3x from transgenic Balb/C (activated with anti-CD3 for 2, 6, 24 h pooled (T203); T cells, highly TH1 polarized 3x from transgenic C57 bl/6 (activated with anti-CD3 for 2, 6, 24 h pooled; T212); T cells, highly TH2 polarized 3x from transgenic C57 bl/6 (activated with anti-CD3 for 2, 6, 24 h pooled; T213); T cells, highly TH1 polarized (naive CD4+ T cells from transgenic Balb/C, polarized 3x with IFNγ, IL-12, and anti-IL-4; stimulated with IGIF, IL-12, and anti IL-4 for 6, 12, 24 h, pooled); CD44- CD25+ pre T cells, sorted from thymus (T204); TH1 T cell clone D1.1, resting for 3 weeks after last stimulation with antigen (T205); TH1 T cell clone D1.1, 10 µg/ml ConA stimulated 15 h (T206); TH2 T cell clone CDC35, resting for 3 weeks after last stimulation with antigen (T207); TH2 T cell clone CDC35, 10 µg/ml ConA stimulated 15 h (T208); unstimulated B cell line CH12 (B201); unstimulated mature B cell leukemia cell line A20 (B200); unstimulated large B cells from spleen (B202); B cells from total spleen, LPS activated (B203); metrizamide enriched dendritic cells from spleen, resting (D200); dendritic cells from bone marrow, resting (D201); unstimulated bone marrow derived dendritic cells depleted with anti B220, anti CD3, and anti Class II, cultured in GM-CSF and IL-4 (D202); bone marrow derived dendritic cells depleted with anti B220, anti CD3, and anti Class II, cultured in GM-CSF and IL-4, stimulated with anti CD40 for 1, 5 d, pooled (D203); monocyte cell line RAW 264.7 activated with LPS 4 h (M200); bone-marrow macrophages derived with GM and M-CSF (M201); bone-marrow macrophages derived with GM-CSF, stimulated with LPS, IFNγ, and IL-10 for 24 h (M205); bone-marrow macrophages derived with GM-CSF, stimulated with LPS, IFNγ, and anti IL-10 for 24 h (M206); peritoneal macrophages (M207); macrophage cell line J774, resting (M202); macrophage cell line J774 + LPS + anti-IL-10 at 0.5, 1, 3, 6, 12 h pooled (M203); macrophage cell line J774 + LPS + IL-10 at 0.5, 1, 3, 5, 12 h pooled (M204); unstimulated mast cell lines MC-9 and MCP-12 (M208); immortalized endothelial cell line derived from brain microvascular endothelial cells, unstimulated (E200); immortalized endothelial cell line derived from brain microvascular endothelial cells, stimulated overnight with TNFα (E201); immortalized endothelial cell line derived from brain microvascular endothelial cells, stimulated overnight with TNFα (E202); immortalized endothelial cell line derived from brain microvascular endothelial cells, stimulated overnight with TNFα and IL-10 (E203); total aorta from wt C57 bl/6 mouse; total aorta from 5 month ApoE KO mouse (X207); total aorta from 12 month ApoE KO mouse (X207); wt thymus (O214); total thymus, rag-1 (0208); total kidney, rag-1 (0209); total kidney, NZ B/W mouse; and total heart, rag-1 (O202). High signal was detected in the monocyte cell line RAW 264.7 activated with LPS 4 h (M200); T cells, highly TH1 polarized 3x from transgenic C57 bl/6 (activated with anti-CD3 for 2, 6, 24 h pooled; T212); and T cells, highly TH1 polarized (naive CD4+ T cells from transgenic Balb/C, polarized 3x with IFNγ, IL-12, and anti-IL-4; stimulated with IGIF, IL-12, and anti IL-4 for 6, 12, 24 h, pooled).

### IV. Chromosome mapping of IL-D80

An isolated cDNA encoding the IL-D80 is used. Chromosome mapping is a standard technique. See, e.g., BIOS Laboratories (New Haven, CT) and methods for using a mouse somatic cell hybrid panel with PCR.

### V. Purification of IL-D80 Protein

Multiple transfected cell lines are screened for one which expresses the cytokine at a high level compared with other cells. Various cell lines are screened and selected for their favorable properties in handling. Natural IL-D80 can be isolated from natural sources, or by expression from a transformed cell using an appropriate expression vector. Purification of the expressed protein is achieved by standard procedures, or may be combined with engineered means for effective purification at high efficiency from cell lysates or supernatants. FLAG or His₆ segments can be used for such purification features. Alternatively, affinity chromatography may be used with specific antibodies, see below.

Protein is produced in coli, insect cell, or mammalian expression systems, as desired. An IL-D80 construct was perpared with an epitope tag extension, e.g., FLAG. The construct was transiently expressed in 293 cells and purified from supernatant using tag immunoaffinity column techniques. Highly purified protein resulted.

### VI. Isolation of Homologous IL-D80 Genes

The IL-D80 cDNA, or other species counterpart sequence, can be used as a hybridization probe to screen a library from a desired source, e.g., a primate cell cDNA library. Many different species can be screened both for stringency necessary for easy hybridization, and for presence using a probe. Appropriate hybridization conditions will be used to select for clones exhibiting specificity of cross hybridization.

Screening by hybridization using degenerate probes based upon the peptide sequences will also allow isolation of appropriate clones. Alternatively, use of appropriate primers for PCR screening will yield enrichment of appropriate nucleic acid clones.

Similar methods are applicable to isolate either species, polymorphic, or allelic variants. Species variants are isolated using cross-species hybridization techniques based upon isolation of a full length isolate or fragment from one species as a probe.

Alternatively, antibodies raised against human IL-D80 will be used to screen for cells which express cross-reactive proteins from an appropriate, e.g., cDNA library. The purified protein or defined peptides are useful for generating antibodies by standard methods, as described above. Synthetic peptides or purified protein are presented to an immune system to generate monoclonal or polyclonal antibodies. See, e.g., Coligan (1991) Current Protocols in Immunology Wiley/Greene; and Harlow and Lane (1989) Antibodies: A Laboratory Manual Cold Spring Harbor Press. The resulting antibodies are used for screening, purification, or diagnosis, as described.

### VII. Preparation of antibodies specific for IL-D80

Synthetic peptides or purified protein are presented to an immune system to generate monoclonal or polyclonal antibodies. See, e.g., Coligan (1991) Current Protocols in Immunology Wiley/Greene; and Harlow and Lane (1989) Antibodies: A Laboratory Manual Cold Spring Harbor Press. Polyclonal serum, or hybridomas may be prepared. In appropriate situations, the binding reagent is either labeled as described above, e.g., fluorescence or otherwise, or immobilized to a substrate for panning methods. Immunoselection, absorptions, and related techniques are available to prepare selective reagents, e.g., exhibiting the desired spectrum of selectivity for binding.

### VIII. Evaluation of Breadth of Biological Functions

Biological activities of IL-D80 are tested, based, in part, on the sequence and structural homology between IL-D80 and IL-11. Initially, assays that show biological activities of IL-11 are examined. See, e.g., Jacobsen (1998) in Thomson The Cytokine Handbook Academic Press.

### A. Effects on proliferation/differentiation of progenitor cells

The effect on proliferation or differentiation of various cell types are evaluated with various concentrations of cytokine. A dose response analysis is performed, in certain cases in combination with other cytokines, e.g., those which synergize with the related cytokine IL-11. These include, e.g., IL-1, IL-4, IL-6, IL-12, LIF, G-CSF, M-CSF, GM-CSF, IL-3, TPO, Kit ligand, or Flt ligand.

In particular, IL-11 exhibits synergistic activities on stem cells. The IL-D80 will be tested on cord blood cells to see if it has effects on proliferation or differentiation of early progenitor cells derived therefrom. Preferably, the cells are early precursor cells, e.g., stem cells, originating from, e.g., cord blood, bone marrow, thymus, spleen, or CD34+ progenitor cells. The cytokine will be tested for effects on myeloid and/or erythroid precursors, including B cell precursors.

### B. Effects of IL-D80 on proliferation of megakaryocytes

Total PBMC are isolated from buffy coats of normal healthy donors by centrifugation through ficoll-hypaque as described (Boyum, et al.). PBMC are cultured in 200 µl Yssel's medium (Gemini Bioproducts, Calabasas, CA) containing 1% human AB serum in 96 well plates (Falcon, Becton-Dickinson, NJ) in the absence or presence of IL-D80, alone or in combination with other cytokines. Cells are cultured in medium alone or in combination with 100 U/ml IL-2 (R&D Systems) for 120 hours. 3H-Thymidine (0.1 mCi) is added during the last six hours of culture and 3H-Thymidine incorporation determined by liquid scintillation counting.

The native, recombinant, and fusion proteins would be tested for agonist and antagonist activity in many other biological assay systems, e.g., on T-cells, B-cells, NK, macrophages, dendritic cells, hematopoietic progenitors, etc.

IL-D80 is evaluated for agonist or antagonist activity on transfected cells expressing IL-11 receptor and controls.

IL-D80 is evaluated for effects, alone or in combination with other cytokines, in macrophage/dendritic cell activation and antigen presentation assays, T cell cytokine production and proliferation in response to antigen or allogeneic stimulus. See, e.g., de Waal Malefyt et al. (1991) J. Exp. Med. 174:1209-1220; de Waal Malefyt et al. (1991) J. Exp. Med. 174:915-924; Fiorentino, et al. (1991) J. Immunol. 147, 3815-3822; Fiorentino, et al. (1991) J. Immunol. 146:3444-3451; and Groux, et al. (1996) J. Exp. Med. 184:19-29.

IL-D80 will also be evaluated for effects on NK cell stimulation. Assays may be based, e.g., on Hsu, et al. (1992) Internat. Immunol. 4:563-569; and Schwarz, et al. (1994) J. Immunother. 16:95-104. Other assays are applied to evaluate effects on cytotoxic T cells and LAK cells. See, e.g., Namien and Mire-Sluis (1998).

B cell growth and differentiation effects will be analyzed, e.g., by the methodology described, e.g., in Defrance, et al. (1992). J. Exp. Med. 175:671-682; Rousset, et al. (1992) Proc. Nat'l Acad. Sci. USA 89:1890-1893; including IgG2 and IgA2 switch factor assays. Note that, unlike COS7 supernatants, NIH3T3 and COP supernatants apparently do not interfere with human B cell assays.

### C. Effects on the expression of cell surface molecules on human monocytes

Monocytes are purified by negative selection from peripheral blood mononuclear cells of normal healthy donors. Briefly, 3 x 10⁸ ficoll banded mononuclear cells are incubated on ice with a cocktail of monoclonal antibodies (Becton-Dickinson; Mountain View, CA) consisting, e.g., of 200 µl of αCD2 (Leu-5A), 200 µl of αCD3 (Leu-4), 100 µl of αCD8 (Leu 2a), 100 µl of αCD19 (Leu-12), 100 µl of αCD20 (Leu-16), 100 µl of αCD56 (Leu-19), 100 µl of αCD67 (IOM 67; Immunotech, Westbrook, ME), and anti-glycophorin antibody (10F7MN, ATCC, Rockville, MD). Antibody bound cells are washed and then incubated with sheep anti-mouse IgG coupled magnetic beads (Dynal, Oslo, Norway) at a bead to cell ratio of 20:1. Antibody bound cells are separated from monocytes by application of a magnetic field. Subsequently, human monocytes are cultured in Yssel's medium (Gemini Bioproducts, Calabasas, CA) containing 1% human AB serum in the absence or presence of IL-D80, alone or in combination with other cytokines.

Analyses of the expression of cell surface molecules can be performed by direct immunofluorescence. For example, 2 x 10⁵ purified human monocytes are incubated in phosphate buffered saline (PBS) containing 1% human serum on ice for 20 minutes. Cells are pelleted at 200 x g. Cells are resuspended in 20 ml PE or FITC labeled mAb. Following an additional 20 minute incubation on ice, cells are washed in PBS containing 1% human serum followed by two washes in PBS alone. Cells are fixed in PBS containing 1% paraformaldehyde and analyzed on FACScan flow cytometer (Becton Dickinson; Mountain View, CA). Exemplary mAbs are used, e.g.: CD11b (anti-macl), CD11c (anti-gp150/95), CD14 (Leu-M3), CD54 (Leu 54), CD80 (anti-BB1/B7), HLA-DR (L243) from Becton-Dickinson and CD86 (FUN 1; Pharmingen), CD64 (32.2; Medarex), CD40 (mAb89; Schering-Plough France).

### D. Effects of IL-D80 on cytokine production by human monocytes

Human monocytes are isolated as described and cultured in Yssel's medium (Gemini Bioproducts, Calabasas, CA) containing 1% human AB serum in the absence or presence of IL-D80 (1/100 dilution baculovirus expressed material). In addition, monocytes are stimulated with LPS (E. coli 0127:B8 Difco) in the absence or presence of IL-D80 and the concentration of cytokines (IL-1β, IL-6, TNFα, GM-CSF, and IL-10) in the cell culture supernatant determined by ELISA.

For intracytoplasmic staining for cytokines, monocytes are cultured (1 million/ml) in Yssel's medium in the absence or presence of IL-D80 and LPS (E. coli 0127:B8 Difco) and 10 mg/ml Brefeldin A (Epicentre technologies Madison WI) for 12 hrs. Cells are washed in PBS and incubated in 2% formaldehyde/PBS solution for 20 minutes at RT. Subsequently cells are washed, resuspended in permeabilization buffer (0.5% saponin (Sigma) in PBS/BSA (0.5%) /Azide (1 mM)) and incubated for 20 minutes at RT. Cells (2 x 10⁵) are centrifuged and resuspended in 20 ml directly conjugated anti-cytokine mAbs diluted 1:10 in permeabilization buffer for 20 minutes at RT. The following antibodies can be used: IL-1α-PE (364-3B3-14); IL-6-PE (MQ2-13A5); TNFα-PE (MAb11); GM-CSF-PE (BVD2-21C11); and IL-12-PE (C11.5.24; Pharmingen San Diego, CA). Subsequently, cells are washed twice in permeabilization buffer and once in PBS/BSA/Azide and analyzed on FACScan flow cytometer (Becton Dickinson; Mountain View, CA).

Additional assays will be tested in the areas of bone remodeling, chondriocytes, neurons, adipocytes, gastrointestinal epithelium, or bronchial epithelium.

### IX. Generation and Analysis of Genetically Altered Animals

Transgenic mice can be generated by standard methods. Such animals are useful to determine the effects of deletion of the gene, in specific tissues, or completely throughout the organism. Such may provide interesting insight into development of the animal or particular tissues in various stages. Moreover, the effect on various responses to biological stress can be evaluated. See, e.g., Hogan, et al. (1995) Manioulatinq the Mouse Embryo: A Laboratory Manual (2d ed.) Cold Spring Harbor Laboratory Press.

Further embodiments of the present invention are disclosed in the following clauses labelled E1 to E20.
E1. An isolated or recombinant polynucleotide encoding an antigenic polypeptide comprising at least 17 contiguous amino acids from the mature polypeptide from SEQ ID NO: 2, 4, 8, or 10.
E2. The polynucleotide of clause E1, encoding a mature polypeptide from SEQ ID NO: 2, 4, 8, or 10.
E3. The polynucleotide of clause E1, which hybridizes at 55° C, less than 500 mM salt, and 50% formamide to the coding portions of SEQ ID NO: 1, 3, 7, or 9.
E4. The polynucleotide of clause E3, comprising at least 35 contiguous nucleotides of the coding portion of SEQ ID NO: 1, 3, 7, or 9.
E5. An expression vector comprising the polynucleotide of clause E1.
E6. A host cell containing the expression vector of clause E5, including a eukaryotic cell.
E7. A method of making an antigenic polypeptide comprising expressing a recombinant polynucleotide of clause E1.
E8. A method for forming a duplex with a polynucleotide of clause E1, comprising contacting said polynucleotide with a probe that hybridizes, under stringent conditions, to at least 25 contiguous nucleotides of the coding portion of SEQ ID NO: 1, 3, 7, or 9; thereby forming said duplex.
E9. A kit for the detection of a polynucleotide of clause E1, comprising a polynucleotide that hybridizes, under stringent hybridization conditions, to at least 17 contiguous nucleotides of a polynucleotide of clause E1.
E10. The kit of clause E9, wherein said probe is detectably labeled.
E11. A binding compound comprising an antibody binding site which specifically binds to at least 17 contiguous amino acids from SEQ ID NO: 2, 4, 8, or 10.
E12. The binding compound of clause E11, wherein:
   a) said antibody binding site is:
      1) specifically immunoreactive with a polypeptide of SEQ ID NO: 2, 4, 8, or 10;
      2) raised against a purified or recombinantly produced human IL-D80 protein; or
      3) in a monoclonal antibody, Fab, or F(ab)2; or
   b) said binding compound is:
      1) an antibody molecule;
      2) a polyclonal antiserum;
      3) detectably labeled;
      4) sterile; or
      5) in a buffered composition.
E13. A method using the binding compound of clause E11, comprising contacting said binding compound with a biological sample comprising an antigen, wherein said contacting results in formation of a binding compound:antigen complex.
E14. The method of clause E13, wherein said biological sample is from a human, and wherein said binding compound is an antibody.
E15. A detection kit comprising said binding compound of clause E12, and:
   a) instructional material for the use of said binding compound for said detection; or
   b) a compartment providing segregation of said binding compound.
E16. A substantially pure or isolated antigenic polypeptide, which binds to said binding composition of clause E11, and further comprises at least 17 contiguous amino acids from SEQ ID NO: 2, 4, 8, or 10.
E17. The polypeptide of clause E16, which:
   a) comprises at least a fragment of at least 25 contiguous amino acid residues from a primate IL-D80 protein;
   b) is a soluble polypeptide;
   c) is detectably labeled;
   d) is in a sterile composition;
   e) is in a buffered composition;
   f) binds to a cell surface receptor;
   g) is recombinantly produced; or
   h) has a naturally occurring polypeptide sequence.
E18. The polypeptide of clause E17, which comprises at least 17 contiguous amino acids of SEQ ID NO: 2, 4, 8, or 10.
E19. A method of modulating physiology or development of a cell or tissue culture cells comprising contacting said cell with an agonist or antagonist of a primate IL-D80.
E20. The method of clause E19, wherein:
   a) said contacting is in combination with an agonist or antagonist of IL-11; or
   b) said contacting is with an antagonist, including binding composition comprising an antibody binding site which specifically binds an IL-D80.

## Claims

1. A method for making an antibody or binding fragment thereof which:
1) specifically binds to the mature polypeptide of SEQ ID NO: 2; or
2) specifically binds to the mature polypeptide of SEQ ID NO: 4; or
3) specifically binds to the mature polypeptide of SEQ ID NO: 8; or
4) specifically binds to the mature polypeptide of SEQ ID NO: 10;
comprising immunizing a non-human host animal with said polypeptide.

2. The method of claim 1 further comprising collecting polyclonal sera from the non-human host animal.

3. The method of claim 1 further comprising generating a hybridoma from said non-human host animal.

4. The method of claim 1 wherein the polypeptide is conjugated to an immunogen.

5. The method of claim 4 wherein the immunogen is keyhole limpet hemocyanin, bovine serum albumin or tetanus toxoid.

6. The method of claim 1 wherein the non-human host animal is a mouse.

7. The method of claim 6 wherein the mouse is a Balb/c mouse.

8. The method of claim 1 wherein the antibody or binding fragment thereof specifically binds to the mature polypeptide of SEQ ID NO: 2.

9. The method of claim 1 wherein the antibody or binding fragment thereof specifically binds to the mature polypeptide of SEQ ID NO: 4.

10. The method of claim 1 wherein the antibody or binding fragment thereof specifically binds to the mature polypeptide of SEQ ID NO: 8.

11. The method of claim 1 wherein the antibody or binding fragment thereof specifically binds to the mature polypeptide of SEQ ID NO: 10.

12. The method of claim 1 wherein the antibody or binding fragment thereof specifically binds to a polypeptide comprising the amino acid sequence set forth in SEQ ID NO: 2.

13. The method of claim 1 wherein the antibody or binding fragment thereof specifically binds to a polypeptide comprising the amino acid sequence set forth in SEQ ID NO: 4.

14. The method of claim 1 wherein the antibody or binding fragment thereof specifically binds to a polypeptide comprising the amino acid sequence set forth in SEQ ID NO: 8.

15. The method of claim 1 wherein the antibody or binding fragment thereof specifically binds to a polypeptide comprising the amino acid sequence set forth in SEQ ID NO: 10.

16. The method of claim 1 wherein the antibody or binding fragment is an antibody.

17. The method of claim 16 wherein the antibody is a monoclonal antibody.

18. The method of claim 1 wherein the antibody or binding fragment thereof comprises a recombinant immunoglobulin.

19. The method of claim 1 wherein the antibody or binding fragment thereof is a Fv fragment.

20. The method of claim 1 wherein the antibody or binding fragment thereof is a Fab fragment.

21. The method of claim 1 wherein the antibody or binding fragment thereof is a F(ab)₂ fragment.

22. The method of claim 1 wherein the antibody or binding fragment thereof is a polyclonal antibody.

23. The method of claim 1 wherein the antibody or binding fragment thereof is detectably labeled.

24. The method of claim 1 for making an antibody that specifically binds to the mature polypeptide of SEQ ID NO: 2 comprising immunizing a non-human host animal with said polypeptide and generating a hybridoma from said non-human host animal.

25. The method of claim 1 for making an antibody that specifically binds to the mature polypeptide of SEQ ID NO: 6 comprising immunizing a non-human host animal with said polypeptide and generating a hybridoma from said non-human host animal.

26. The method of claim 1 for making an antibody that specifically binds to the mature polypeptide of SEQ ID NO: 10 comprising immunizing a non-human host animal with said polypeptide and generating a hybridoma from said non-human host animal.

## Patentansprüche

1. Ein Verfahren zur Herstellung eines Antikörpers oder eines bindenden Fragments davon, der/das:
1) spezifisch an das reife Polypeptid der SEQ ID NO: 2 bindet oder
2) spezifisch an das reife Polypeptid der SEQ ID NO: 4 bindet oder
3) spezifisch an das reife Polypeptid der SEQ ID NO: 8 bindet oder
4) spezifisch an das reife Polypeptid der SEQ ID NO: 10 bindet,
umfassend das Immunisieren eines nichtmenschlichen Wirtstieres mit dem Polypeptid

2. Das Verfahren nach Anspruch 1, das ferner die Entnahme polyklonaler Sera vom nichtmenschlichen Wirtstier umfasst.

3. Das Verfahren nach Anspruch 1, das ferner das Erzeugen eines Hybridoms aus dem nichtmenschlichen Wirtstier umfasst.

4. Das Verfahren nach Anspruch 1, wobei das Polypeptid an ein Immunogen konjugiert ist.

5. Das Verfahren nach Anspruch 4, wobei das Immunogen Schlitzschnecken-Hämocyanin, bovines Serumalbumin oder Tetanus-Toxoid ist.

6. Das Verfahren nach Anspruch 1, wobei das nichtmenschliche Wirtstier eine Maus ist.

7. Das Verfahren nach Anspruch 6, wobei die Maus eine Balb/c-Maus ist.

8. Das Verfahren nach Anspruch 1, wobei der Antikörper oder das bindende Fragment davon spezifisch an das reife Polypeptid der SEQ ID NO: 2 bindet.

9. Das Verfahren nach Anspruch 1, wobei der Antikörper oder das bindende Fragment davon spezifisch an das reife Polypeptid der SEQ ID NO: 4 bindet.

10. Das Verfahren nach Anspruch 1, wobei der Antikörper oder das bindende Fragment davon spezifisch an das reife Polypeptid der SEQ ID NO: 8 bindet.

11. Das Verfahren nach Anspruch 1, wobei der Antikörper oder das bindende Fragment davon spezifisch an das reife Polypeptid der SEQ ID NO: 10 bindet.

12. Das Verfahren nach Anspruch 1, wobei der Antikörper oder das bindende Fragment davon spezifisch an ein Polypeptid bindet, das die in SEQ ID NO: 2 angegebene Aminosäuresequenz umfasst.

13. Das Verfahren nach Anspruch 1, wobei der Antikörper oder das bindende Fragment davon spezifisch an ein Polypeptid bindet, das die in SEQ ID NO: 4 angegebene Aminosäuresequenz umfasst.

14. Das Verfahren nach Anspruch 1, wobei der Antikörper oder das bindende Fragment davon spezifisch an ein Polypeptid bindet, das die in SEQ ID NO: 8 angegebene Aminosäuresequenz umfasst.

15. Das Verfahren nach Anspruch 1, wobei der Antikörper oder das bindende Fragment davon spezifisch an ein Polypeptid bindet, das die in SEQ ID NO: 10 angegebene Aminosäuresequenz umfasst.

16. Das Verfahren nach Anspruch 1, wobei der Antikörper oder das bindende Fragment ein Antikörper ist.

17. Das Verfahren nach Anspruch 16, wobei der Antikörper ein monoklonaler Antikörper ist.

18. Das Verfahren nach Anspruch 1, wobei der Antikörper oder das bindende Fragment davon ein rekombinantes Immunglobulin umfasst.

19. Das Verfahren nach Anspruch 1, wobei der Antikörper oder das bindende Fragment davon ein Fv-Fragment ist.

20. Das Verfahren nach Anspruch 1, wobei der Antikörper oder das bindende Fragment davon ein Fab-Fragment ist.

21. Das Verfahren nach Anspruch 1, wobei der Antikörper oder das bindende Fragment davon ein F(ab)₂-Fragment ist.

22. Das Verfahren nach Anspruch 1, wobei der Antikörper oder das bindende Fragment davon ein polyklonaler Antikörper ist.

23. Das Verfahren nach Anspruch 1, wobei der Antikörper oder das bindende Fragment davon nachweisbar markiert ist.

24. Das Verfahren nach Anspruch 1 zur Herstellung eines Antikörpers, der spezifisch an das reife Polypeptid der SEQ ID NO: 2 bindet, umfassend das Immunisieren eines nichtmenschlichen Wirtstieres mit dem Polypeptid und das Erzeugen eines Hybridoms aus dem nichtmenschlichen Wirtstier.

25. Das Verfahren nach Anspruch 1 zur Herstellung eines Antikörpers, der spezifisch an das reife Polypeptid der SEQ ID NO: 6 bindet, umfassend das Immunisieren eines nichtmenschlichen Wirtstieres mit dem Polypeptid und das Erzeugen eines Hybridoms aus dem nichtmenschlichen Wirtstier.

26. Das Verfahren nach Anspruch 1 zur Herstellung eines Antikörpers, der spezifisch an das reife Polypeptid der SEQ ID NO: 10 bindet, umfassend das Immunisieren eines nichtmenschlichen Wirtstieres mit dem Polypeptid und das Erzeugen eines Hybridoms aus dem nichtmenschlichen Wirtstier.

## Revendications

1. Méthode pour la fabrication d'un anticorps ou d'un fragment de liaison de celui-ci qui:
1) se lie spécifiquement au polypeptide mature de la SEQ ID NO:2; ou
2) se lie spécifiquement au polypeptide mature de la SEQ ID NO:4; ou
3) se lie spécifiquement au polypeptide mature de la SEQ ID NO:8; ou
4) se lie spécifiquement au polypeptide mature de la SEQ ID NO:10;
comprenant l'immunisation d'un animal hôte non humain avec ledit polypeptide.

2. Méthode selon la revendication 1, comprenant en outre la récupération de sérums polyclonaux à partir de l'animal hôte non humain.

3. Méthode selon la revendication 1, comprenant en outre la génération d'un hybridome à partir dudit animal hôte non humain.

4. Méthode selon la revendication 1, dans laquelle le polypeptide est conjugué à un immunogène.

5. Méthode selon la revendication 4, dans laquelle l'immunogène est une hémocyanine de patelle, une albumine de sérum bovin ou un toxoïde tétanique.

6. Méthode selon la revendication 1, dans laquelle l'animal hôte non humain est une souris.

7. Méthode selon la revendication 6, dans laquelle la souris est une souris Balb/c.

8. Méthode selon la revendication 1, dans laquelle l'anticorps ou le fragment de liaison de celui-ci se lie spécifiquement au polypeptide mature de la SEQ ID NO:2.

9. Méthode selon la revendication 1, dans laquelle l'anticorps ou le fragment de liaison de celui-ci se lie spécifiquement au polypeptide mature de la SEQ ID NO:4.

10. Méthode selon la revendication 1, dans laquelle l'anticorps ou le fragment de liaison de celui-ci se lie spécifiquement au polypeptide mature de la SEQ ID NO:8.

11. Méthode selon la revendication 1, dans laquelle l'anticorps ou le fragment de liaison de celui-ci se lie spécifiquement au polypeptide mature de la SEQ ID NO:10.

12. Méthode selon la revendication 1, dans laquelle l'anticorps ou le fragment de liaison de celui-ci se lie spécifiquement à un polypeptide comprenant la séquence d'acides aminés présentée dans la SEQ ID NO:2.

13. Méthode selon la revendication 1, dans laquelle l'anticorps ou le fragment de liaison de celui-ci se lie spécifiquement à un polypeptide comprenant la séquence d'acides aminés présentée dans la SEQ ID NO:4.

14. Méthode selon la revendication 1, dans laquelle l'anticorps ou le fragment de liaison de celui-ci se lie spécifiquement à un polypeptide comprenant la séquence d'acides aminés présentée dans la SEQ ID NO:8.

15. Méthode selon la revendication 1, dans laquelle l'anticorps ou le fragment de liaison de celui-ci se lie spécifiquement à un polypeptide comprenant la séquence d'acides aminés présentée dans la SEQ ID NO:10.

16. Méthode selon la revendication 1, dans laquelle l'anticorps ou le fragment de liaison est un anticorps.

17. Méthode selon la revendication 16, dans laquelle l'anticorps est un anticorps monoclonal.

18. Méthode selon la revendication 1, dans laquelle l'anticorps ou le fragment de liaison de celui-ci comprend une immunoglobuline recombinante.

19. Méthode selon la revendication 1, dans laquelle l'anticorps ou le fragment de liaison de celui-ci est un fragment Fv.

20. Méthode selon la revendication 1, dans laquelle l'anticorps ou le fragment de liaison de celui-ci est un fragment Fab.

21. Méthode selon la revendication 1, dans laquelle l'anticorps ou le fragment de liaison de celui-ci est un fragment F(ab)₂.

22. Méthode selon la revendication 1, dans laquelle l'anticorps ou le fragment de liaison de celui-ci est un anticorps polyclonal.

23. Méthode selon la revendication 1, dans laquelle l'anticorps ou le fragment de liaison de celui-ci est marqué de façon détectable.

24. Méthode selon la revendication 1 pour la fabrication d'un anticorps qui se lie spécifiquement au polypeptide mature de la SEQ ID NO:2 comprenant l'immunisation d'un animal hôte non humain avec ledit polypeptide et la génération d'un hybridome à partir dudit animal hôte non humain.

25. Méthode selon la revendication 1 pour la fabrication d'un anticorps qui se lie spécifiquement au polypeptide mature de la SEQ ID NO:6 comprenant l'immunisation d'un animal hôte non humain avec ledit polypeptide et la génération d'un hybridome à partir dudit animal hôte non humain.

26. Méthode selon la revendication 1 pour la fabrication d'un anticorps qui se lie spécifiquement au polypeptide mature de la SEQ ID NO:10 comprenant l'immunisation d'un animal hôte non humain avec ledit polypeptide et la génération d'un hybridome à partir dudit animal hôte non humain.
